(19) Europäisches Patentamt European Patent Office Office européen des brevets

(11) EP 4 781 905 A1

(12) **EUROPEAN PATENT APPLICATION**
published in accordance with Art. 153(4) EPC

(43) Date of publication:
29.07.2026 Bulletin 2026/31

(21) Application number: 24878602.2

(22) Date of filing: 26.07.2024

(51) International Patent Classification (IPC):
*A61B 5/024* (2006.01)

(52) Cooperative Patent Classification (CPC):
A61B 5/021; A61B 5/024; A61B 5/1455

(86) International application number:
PCT/CN2024/107911

(87) International publication number:
WO 2025/081950 (24.04.2025 Gazette 2025/17)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 16.10.2023 CN 202311344325

(71) Applicant: Honor Device Co., Ltd.
Shenzhen, Guangdong 518040 (CN)

(72) Inventors:
• LI, Chenlong
  Shenzhen, Guangdong 518040 (CN)
• JIA, Xingwang
  Shenzhen, Guangdong 518040 (CN)
• ZHANG, Jicheng
  Shenzhen, Guangdong 518040 (CN)

(74) Representative: Gill Jennings & Every LLP
The Broadgate Tower
20 Primrose Street
London EC2A 2ES (GB)

(54) **TEST CIRCUIT AND METHOD, AND ELECTRONIC DEVICE**

(57) This application provides a test circuit and method, and an electronic device. The test circuit has a small volume and low costs, which is conducive to miniaturization of the electronic device, and is conducive to reducing costs of the electronic device. The test circuit includes: a light-emitting assembly, configured to emit at least two types of optical signals, where the at least two types of optical signals include a first optical signal and a second optical signal; and the first optical signal and the second optical signal have different wavelengths; and an optical proximity sensor, configured to collect a first optical reference signal and a second optical reference signal that are reflected and/or scattered by a human body, where the first optical reference signal and the second optical reference signal are used for calculating a human body feature value.

FIG. 12

## Description

This application claims priority to Chinese Patent Application No. 202311344325.7, filed with the China National Intellectual Property Administration on October 16, 2023 and entitled "TEST CIRCUIT AND METHOD, AND ELECTRONIC DEVICE", which is incorporated herein by reference in its entirety.

## TECHNICAL FIELD

This application relates to the field of electronic technologies, and in particular, to a test circuit and method, and an electronic device.

## BACKGROUND

At present, with the development of terminal technologies, terminal devices have become a part of people's work and life. To satisfy requirements of users for health management thereof, a large quantity of terminal devices can support a function of monitoring vital sign parameters of the users. For example, a user may measure human body features such as a heart rate, a breathing rate, blood pressure, or blood oxygen of a human body by using a wearable device such as a smart watch. Usually, a terminal device may be configured with a photoplethysmograph (photoplethysmograph, PPG) module for measuring human body features. However, an existing PPG module has a problem of large space occupation.

## SUMMARY

To resolve the foregoing technical problem, this application provides a test circuit and method, and an electronic device. The test circuit in this application has a small volume and low costs, which is conducive to miniaturization of the electronic device, and is conducive to reducing costs of the electronic device.

According to a first aspect, an embodiment of this application provides a test circuit. The test circuit includes: a light-emitting assembly, configured to emit at least two types of optical signals, where the at least two types of optical signals include a first optical signal and a second optical signal; and the first optical signal and the second optical signal have different wavelengths; and an optical proximity sensor, configured to collect a first optical reference signal and a second optical reference signal that are reflected and/or scattered by a human body, where the first optical reference signal and the second optical reference signal are used for calculating a human body feature value.

The light-emitting assembly and the optical proximity sensor are used to replace an existing PPG module. Because the optical proximity sensor is widely used and has a low price, costs can be reduced when a PPG signal is detected by using the optical proximity sensor in the test circuit provided in this embodiment of this application. In addition, because structures for collecting an optical signal and processing the optical signal are integrated in the optical proximity sensor, in comparison with the PPG module in the related art, a volume of the test circuit that can measure a PPG signal in this embodiment of this application is smaller.

For example, the at least two types of optical signals may alternatively include the first optical signal, the second optical signal, and a third optical signal, or the at least two types of optical signals may include the first optical signal, the second optical signal, a third optical signal, and a fourth optical signal, or the at least two types of optical signals may include the first optical signal, the second optical signal, a third optical signal, a fourth optical signal, and a fifth optical signal, or the like. In other words, the test circuit can calculate blood oxygen saturation, a heart rate, and the like based on signals in two different optical bands (for example, the first optical signal and the second optical signal), and can further couple optical signals in other bands to determine the PPG signal, to calculate, based on the PPG signal, human vital sign parameters such as a blood pressure, blood glucose, a perfusion index, a pulse variability index, and content of blood indicators such as carboxyhemoglobin and glycated hemoglobin.

For example, the first optical reference signal reflected and/or scattered by the human body may be the first optical reference signal reflected by the human body, may be the first optical reference signal scattered by the human body, or may be the first optical reference signal reflected and scattered by the human body.

According to the first aspect, the optical proximity sensor includes: a collection unit, configured to: collect the first optical reference signal and the second optical reference signal, and separately convert the first optical reference signal and the second optical reference signal into an electrical signal, where the electrical signal is an analog signal; and an analog-to-digital converter, connected to the collection unit, and configured to convert the analog signal into a digital signal, where the digital signal is used for calculating the human body feature value.

The foregoing is a specific structure of the optical proximity sensor, and an internal structure of the optical proximity sensor is simple, so that a volume of the optical proximity sensor is small. Certainly, the structure of the optical proximity sensor is not limited thereto. Any optical proximity sensor that can collect the first optical reference signal and the second optical reference signal and calculate the human body feature value based on the collected signals shall fall within the protection scope of this application.

According to the first aspect or any implementation of the first aspect, the light-emitting assembly includes a first light-emitting unit and a second light-emitting unit, the collection unit includes a first photosensitive component and a second photosensitive component, the first light-emitting unit is configured to emit the first optical signal, the first photosensitive component is configured to collect the first optical reference signal, the second light-emitting unit is configured to emit the second optical signal, and the second photosensitive component is configured to collect the second optical reference signal.

In other words, different photosensitive components respond to optical signals in different bands (that is, may convert the optical signals in different bands into electrical signals), so that the optical signals do not affect each other, thereby improving detection accuracy.

For example, a quantity of the photosensitive components in the collection unit may be in a one-to-one correspondence with a quantity of the light-emitting units. For example, the light-emitting assembly includes the first light-emitting unit, the second light-emitting unit, and a third light-emitting unit. The first light-emitting unit, the second light-emitting unit, and the third light-emitting unit respectively emit the first optical signal, the second optical signal, and a third optical signal. Correspondingly, the collection unit includes the first photosensitive component, the second photosensitive component, and a third photosensitive component. The first photosensitive component is configured to collect the first optical reference signal, the second photosensitive component is configured to collect the second optical reference signal, and the third photosensitive component is configured to collect a third optical reference signal that is reflected and/or scattered by the human body.

According to the first aspect or any implementation of the first aspect, the optical proximity sensor includes the first light-emitting unit or the second light-emitting unit.

In other words, an emitting light source inside the optical proximity sensor is used as one of the light-emitting units. In this way, the quantity of light-emitting units that need to be disposed in the light-emitting assembly can be reduced, thereby further reducing the volume of the test circuit.

According to the first aspect or any implementation of the first aspect, a processing unit is further included. The processing unit is connected to the analog-to-digital converter and configured to obtain, based on the digital signal, an integral value of a light intensity of the first optical reference signal within a first time period and an integral value of a light intensity of the second optical reference signal within a second time period, and the integral values are used for calculating the human body feature value.

For example, the optical proximity sensor includes the processing unit, in other words, the processing unit may be disposed in the optical proximity sensor. In this way, the processing unit does not need to be disposed externally, thereby further reducing the volume of the test circuit.

For example, the processing unit may alternatively be an external processing unit, that is, a component having a specific processing function in the electronic device, such as a processor. The digital signal is processed by using a component having a processing function in the electronic device, and no processing unit needs to be separately disposed, thereby further reducing the volume of the test circuit and reducing costs of the test circuit.

According to the first aspect or any implementation of the first aspect, the light-emitting assembly emits the first optical signal and the second optical signal according to a duty cycle. In this way, a plurality of groups of first optical reference signals and second optical reference signals may be obtained, and the human body feature value is calculated based on the plurality of groups of first optical reference signals and second optical reference signals.

For example, the processing unit in the optical proximity sensor may drive the light-emitting assembly to emit the first optical signal and the second optical signal according to the duty cycle; or a component having a specific processing function in the electronic device, such as a processor, may drive the light-emitting assembly to emit the first optical signal and the second optical signal according to the duty cycle.

According to the first aspect or any implementation of the first aspect, the duty cycle includes at least a first stage and a second stage; the first stage and the second stage both include at least one pulse cycle, and the pulse cycle includes a light-emitting stage at which working is performed in a first level state and a non-light-emitting stage at which working is performed in a second level state; the light-emitting assembly is configured to emit the first optical signal at the light-emitting stage of the first stage, and emit the second optical signal at the light-emitting stage of the second stage; the optical proximity sensor is configured to collect a third optical signal at the light-emitting stage of the first stage, and collect a fourth optical signal at the non-light-emitting stage of the first stage; and is further configured to collect a fifth optical signal at the light-emitting stage of the second stage, and collect a sixth optical signal at the non-light-emitting stage of the second stage; the processing unit is configured to obtain the integral value of the light intensity of the first optical reference signal based on output of the third optical signal and the fourth optical signal; and is further configured to obtain the integral value of the light intensity of the second optical reference signal based on output of the fifth optical signal and the sixth optical signal; the first time period is the first stage, and the second time period is the second stage; and the third optical signal is determined based on ambient light and reflection and/or scattering of the first optical signal, the fourth optical signal is determined based on the ambient light, the fifth optical signal is determined based on the ambient light and reflection and/or scattering of the second optical signal, and the sixth optical signal is determined based on the ambient light.

The test circuit detects reflected and/or scattered light of the first optical signal and the second optical signal, and detects the ambient light. In this way, interference from the external ambient light can be reduced, background noise can be filtered out, and detection accuracy can be improved.

For example, the first optical reference signal includes the first optical signal and a signal that is obtained after the ambient light is reflected and/or scattered by the human body at the light-emitting stage of the first stage, and includes a signal that is obtained after the ambient light is reflected and/or scattered by the human body at the non-light-emitting stage of the first stage.

For example, the second optical reference signal includes the second optical signal and a signal that is obtained after the ambient light is reflected and/or scattered by the human body at the light-emitting stage of the second stage, and includes a signal that is obtained after the ambient light is reflected and/or scattered by the human body at the non-light-emitting stage of the second stage.

For example, the first level is, for example, a high level, and the second level is, for example, a low level.

For example, that the third optical signal is determined based on the ambient light and reflection and/or scattering of the first optical signal may be that the third optical signal is determined based on the ambient light and the reflection of the first optical signal, may be that the third optical signal is determined based on the ambient light and the scattering of the first optical signal, or may be that the third optical signal is determined based on the ambient light and the reflection and the scattering of the first optical signal.

For example, that the fifth optical signal is determined based on the ambient light and reflection and/or scattering of the second optical signal may be that the fifth optical signal is determined based on the ambient light and the reflection of the second optical signal, may be that the fifth optical signal is determined based on the ambient light and the scattering of the second optical signal, or may be that the fifth optical signal is determined based on the ambient light and the reflection and the scattering of the second optical signal.

According to the first aspect or any implementation of the first aspect, the collection unit includes a first photosensitive component and a second photosensitive component, the first photosensitive component and the analog-to-digital converter form a first collection channel, and the second photosensitive component and the analog-to-digital converter form a second collection channel; the first collection channel is configured to collect the third optical signal at the light-emitting stage of the first stage, and collect the fourth optical signal at the non-light-emitting stage of the first stage; and the second collection channel is configured to collect the fifth optical signal at the light-emitting stage of the second stage, and collect the sixth optical signal at the non-light-emitting stage of the second stage.

Because the analog-to-digital converter is shared, the first collection channel and the second collection channel collect the third optical signal and the fourth optical signal, and the fifth optical signal and the sixth optical signal in time division, to avoid mutual influence between different optical signals.

According to the first aspect or any implementation of the first aspect, the first stage and the second stage both include N pulse cycles, where N≥2, and N is a positive integer.

To be specific, an emission cycle of a single pulse (an optical signal) is divided into several pulse cycles, and pulse duration is shortened, so that impact of a flicker light source can be reduced and anti-interference capability can be improved without affecting sensitivity.

According to the first aspect or any implementation of the first aspect, a duty cycle includes at least one pulse cycle, and the pulse cycle includes a light-emitting stage at which working is performed in a first level state and a non-light-emitting stage at which working is performed in a second level state; the light-emitting assembly is configured to emit the first optical signal and the second optical signal at the light-emitting stage; the optical proximity sensor is configured to collect a seventh optical signal and an eighth optical signal at the light-emitting stage, and collect a ninth optical signal and a tenth optical signal at the non-light-emitting stage; the processing unit is configured to obtain the integral value of the light intensity of the first optical reference signal based on the seventh optical signal and the ninth optical signal; and is further configured to obtain the integral value of the light intensity of the second optical reference signal based on the eighth optical signal and the tenth optical signal; the first time period and the second time period are a same time period, and the first time period is one duty cycle; and the seventh optical signal is determined based on ambient light and reflection and/or scattering of the first optical signal, the eighth optical signal is determined based on the ambient light and reflection and/or scattering of the second optical signal, the ninth optical signal is determined based on the ambient light, and the tenth optical signal is determined based on the ambient light.

The test circuit detects reflected and/or scattered light of the first optical signal and the second optical signal, and detects the ambient light. In this way, interference from the external ambient light can be reduced, background noise can be filtered out, and detection accuracy can be improved. In addition, when the optical proximity sensor includes a plurality of analog-to-digital converters, the optical proximity sensor may simultaneously collect the seventh optical signal and the eighth optical signal at the light-emitting stage, and simultaneously collect the ninth optical signal and the tenth optical signal at the non-light-emitting stage, so that optical signals in different bands are collected at a same moment. In comparison with collecting optical signals in different bands in time division (that is, at different moments), because the vital sign parameters are determined based on the optical signals in different bands, the optical signals in different bands obtained in time division cause a time difference between vital signs of the user, and cannot better reflect real vital signs. In this solution, optical signals in different bands at a same moment can be obtained, that is, the vital signs of the user at the same moment can be reflected, so that the physiological change data of the user is better tracked, and the vital signs can be more truly reflected. Based on this, longer duration can be reserved for the analog-to-digital converter to perform optical signal processing, so that integration detection duration can be improved, and better sensitivity can be achieved.

For example, the first optical reference signal includes both the first optical signal and the signal that is obtained after the ambient light is reflected and/or scattered by the human body at the light-emitting stage, and the signal that is obtained after the ambient light is reflected and/or scattered by the human body at the non-light-emitting stage.

For example, the second optical reference signal includes both the second optical signal and the signal that is obtained after the ambient light is reflected and/or scattered by the human body at the light-emitting stage, and the signal that is obtained after the ambient light is reflected and/or scattered by the human body at the non-light-emitting stage.

For example, the first level is, for example, a high level, and the second level is, for example, a low level.

For example, that the seventh optical signal is determined based on the ambient light and reflection and/or scattering of the first optical signal may be that the seventh optical signal is determined based on the ambient light and the reflection of the first optical signal, may be that the seventh optical signal is determined based on the ambient light and the scattering of the first optical signal, or may be that the seventh optical signal is determined based on the ambient light and the reflection and the scattering of the first optical signal.

For example, that the eighth optical signal is determined based on the ambient light and reflection and/or scattering of the second optical signal may be that the eighth optical signal is determined based on the ambient light and the reflection of the second optical signal, may be that the eighth optical signal is determined based on the ambient light and the scattering of the second optical signal, or may be that the eighth optical signal is determined based on the ambient light and the reflection and the scattering of the second optical signal.

According to the first aspect or any implementation of the first aspect, the collection unit includes a first photosensitive component and a second photosensitive component, and the analog-to-digital converter includes a first analog-to-digital converter and a second analog-to-digital converter; the first photosensitive component and the first analog-to-digital converter form a first collection channel, and the second photosensitive component and the second analog-to-digital converter form a second collection channel; the first collection channel is configured to collect the seventh optical signal at the light-emitting stage, and collect the ninth optical signal at the non-light-emitting stage; and the second collection channel is configured to collect the eighth optical signal at the light-emitting stage, and collect the tenth optical signal at the non-light-emitting stage.

Because there are a plurality of analog-to-digital converters, the first collection channel and the second collection channel may simultaneously collect the seventh optical signal and the eighth optical signal at the light-emitting stage, and simultaneously collect the ninth optical signal and the tenth optical signal at the non-light-emitting stage, thereby avoiding mutual influence between different optical signals.

According to the first aspect or any implementation of the first aspect, the duty cycle includes N pulse cycles, where N≥2, and N is a positive integer.

To be specific, an emission cycle of a single pulse (an optical signal) is divided into several pulse cycles, and pulse duration is shortened, so that impact of a flicker light source can be reduced and anti-interference capability can be improved without affecting sensitivity.

According to the first aspect or any implementation of the first aspect, the first optical signal includes a red light signal, and the second optical signal includes an infrared light signal.

This is conducive to calculation of blood oxygen saturation in blood.

Certainly, this does not constitute a limitation on this application. A person skilled in the art may make selection based on an actual situation. Optionally, the first optical signal may alternatively include, for example, a green light signal, and the second optical signal includes a blue light signal or the like; or the first optical signal may include, for example, a red light signal, and the second optical signal includes a green light signal; or the first optical signal may include, for example, a green light signal, and the second optical signal may include an infrared light signal or the like.

Certainly, an optical signal in another band may alternatively be coupled, to implement different functions, and this is not limited to optical signals in two bands.

According to a second aspect, an embodiment of this application provides an electronic device. The electronic device: the test circuit according to the first aspect and any implementation of the first aspect.

The second aspect and any implementation of the second aspect correspond to the first aspect and any implementation of the first aspect respectively. For technical effects corresponding to the second aspect and any implementation of the second aspect, refer to the technical effects corresponding to the first aspect and any implementation of the first aspect. Details are not described herein again.

According to the second aspect, the electronic device further includes a display screen, and the display screen is reused as the light-emitting assembly.

The display screen in the electronic device is directly used as the light-emitting assembly. In this way, the light-emitting assembly does not need to be separately disposed, thereby reducing costs, further reducing occupation of the electronic device by the test circuit, and releasing more space for disposing another structure.

According to the second aspect or any implementation of the first aspect, the display screen includes a display region and a non-display region surrounding the display region, and the optical proximity sensor is located in the non-display region.

During detection, a finger of a user needs to cover the optical proximity sensor and a part of a display region of the display screen, to facilitate detection of the user.

Certainly, a position of the optical proximity sensor is not limited thereto. Optionally, the optical proximity sensor may alternatively be disposed at a position below the display screen, or the like.

According to the second aspect or any implementation of the first aspect, the electronic device includes a display screen and a flash light that are disposed on opposite sides of the electronic device, where the optical proximity sensor and the flash light are disposed on a same side of the electronic device, the flash light is reused as the light-emitting assembly, and an optical signal emitted by the flash light includes at least the first optical signal and the second optical signal.

The flash light in the electronic device is directly used as the light-emitting assembly. In this way, the light-emitting assembly does not need to be separately disposed, thereby reducing costs, further reducing occupation of the electronic device by the test circuit, and releasing more space for disposing another structure.

The optical proximity sensor is disposed near the flash light, and during detection, the finger of the user needs to cover the optical proximity sensor and the flash light.

According to a third aspect, an embodiment of this application provides a test method. The test method is applied to an electronic device, where the electronic device includes a plurality of duty cycles, the duty cycle includes at least one pulse cycle, and the pulse cycle includes a light-emitting stage at which working is performed in a first level state and a non-light-emitting stage at which working is performed in a second level state; and the test method includes: emitting a first optical signal and a second optical signal, and collecting a seventh optical signal and an eighth optical signal at the light-emitting stage, where the first optical signal and the second optical signal have different wavelengths, the seventh optical signal is determined based on ambient light and reflection and/or scattering of the first optical signal, and the eighth optical signal is determined based on the ambient light and reflection and/or scattering of the second optical signal; collecting a ninth optical signal and a tenth optical signal at the non-light-emitting stage, where the ninth optical signal is determined by the ambient light, and the tenth optical signal is determined by the ambient light; and obtaining an integral value of a light intensity of a first optical reference signal based on the seventh optical signal and the ninth optical signal, and obtaining an integral value of a light intensity of a second optical reference signal based on the eighth optical signal and the tenth optical signal.

The light-emitting assembly may simultaneously emit at least two types of optical signals. The electronic device collects and processes the seventh optical signal and the ninth optical signal, to obtain the integral value of the light intensity of the first optical reference signal. At the same time, the electronic device collects and processes the eighth optical signal and the tenth optical signal, to obtain the integral value of the light intensity of the second optical reference signal, that is, optical signals in different bands are obtained at a same moment. In comparison with obtaining optical signals in different bands in time division (that is, at different moments), because the vital sign parameters are determined based on the optical signals in different bands, the optical signals in different bands obtained in time division cause a time difference between vital signs of the user, and cannot better reflect real vital signs. In this solution, optical signals in different bands at a same moment can be obtained, that is, the vital signs of the user at the same moment can be reflected, so that the physiological change data of the user is better tracked, and the vital signs can be more truly reflected. Based on this, because the optical signals in different bands do not need to be collected in time division, longer duration can be reserved for optical signal processing, so that longer integration duration can be reserved, and better sensitivity can be achieved.

According to a fourth aspect, an embodiment of this application provides an electronic device. The electronic device includes: a memory and a processor, where the memory is coupled to the processor; and the memory stores program instructions, and the program instructions, when executed by the processor, enable the electronic device to perform the test method according to the third aspect.

The fourth aspect corresponds to the third aspect. For technical effects corresponding to the fourth aspect, refer to the technical effects corresponding to the third aspect. Details are not described herein again.

For example, the electronic device includes a mobile phone, a tablet computer, or a wearable device.

Certainly, this does not constitute a limitation on this application. A person skilled in the art may make selection based on an actual situation. Optionally, the electronic device may further include a medical device, a notebook computer, a smart home device, a personal digital assistant (personal digital assistant, PDA for short), an in-vehicle computer, or the like.

## BRIEF DESCRIPTION OF DRAWINGS

FIG. 1 is a diagram of absorption spectra of different types of hemoglobin;
FIG. 2 is a schematic diagram of a structure of an electronic device according to an embodiment of this application;
FIG. 3 is a cross-sectional view of the electronic device shown in FIG. 2 in a direction A-A';
FIG. 4 is a diagram of a working principle of an optical proximity sensor;
FIG. 5 is a schematic diagram of an application scenario of an electronic device according to an embodiment of this application;
FIG. 6 is a circuit diagram of a test circuit according to an embodiment of this application;
FIG. 7 is a top view of a structure of an optical proximity sensor according to an embodiment of this application;
FIG. 8 shows an example of a test circuit according to an embodiment of this application;
FIG. 9 is a sequence diagram of time-division driving of a test circuit according to an embodiment of this application;
FIG. 10 is a diagram of comparison between a working sequence of a light-emitting assembly and a working sequence of a flicker light source according to an embodiment of this application;
FIG. 11 is another sequence diagram of time-division driving of a test circuit according to an embodiment of this application;
FIG. 12 is a circuit diagram of another test circuit according to an embodiment of this application;
FIG. 13 shows an example of an optical proximity sensor according to an embodiment of this application;
FIG. 14 is a sequence diagram of simultaneous driving of a test circuit according to an embodiment of this application;
FIG. 15 is another sequence diagram of simultaneous driving of a test circuit according to an embodiment of this application;
FIG. 16 is a diagram of an interface of a test result of blood oxygen and a heart rate obtained based on a test circuit according to an embodiment of this application; and
FIG. 17 is a diagram that is of oxygen decreasing monitoring and that is obtained based on a test circuit according to an embodiment of this application.

## DESCRIPTION OF EMBODIMENTS

The technical solutions in embodiments of this application are clearly and completely described in the following with reference to the accompanying drawings in embodiments of this application. Apparently, the described embodiments are merely some rather than all of embodiments of this application. All other embodiments obtained by a person of ordinary skill in the art based on embodiments of this application without creative efforts shall fall within the protection scope of this application.

The term "and/or" used in this specification describes only an association relationship for describing associated objects and represents that three relationships may exist. For example, A and/or B may represent the following three cases: Only A exists, both A and B exist, and only B exists.

In the specification of embodiments of this application and claims, the terms "first", "second", and the like are intended to distinguish between different objects but do not indicate a specific order of the objects. For example, a first target object, a second target object, and the like are used to distinguish between different target objects, but are not used to describe a particular sequence of the target objects.

In embodiments of this application, terms such as "exemplary" or "for example" is used to represent giving an example, an illustration, or a description. Any embodiment or design solution described by using "exemplary" or "for example" in embodiments of this application should not be explained as being more preferred or having more advantages than another embodiment or design solution. More exactly, the use of terms such as "exemplary" or "for example" is intended to present concepts in a specific manner.

In the descriptions of embodiments of this application, unless otherwise stated, "a plurality of" means two or more. For example, a plurality of processing units means two or more processing units; and a plurality of systems means two or more systems.

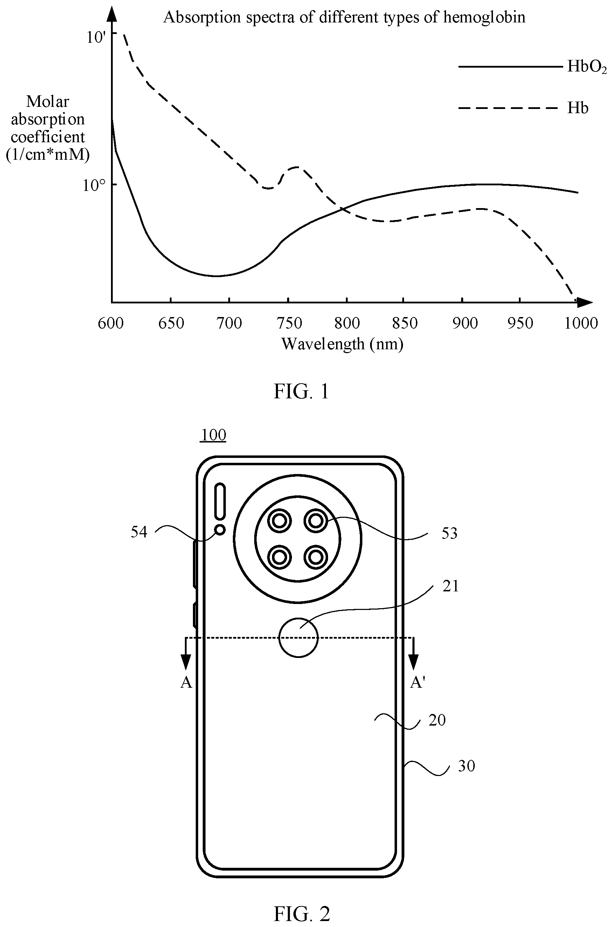

When illumination is transmitted through the skin tissue and then reflected to a detection structure, the illumination is attenuated to some extent. The absorption of light by tissues such as muscles, bones, veins, and other connecting tissues remains basically unchanged (provided that a measurement position does not move significantly). However, blood is different. Because there is blood flowing in arteries, absorption of light by blood naturally varies. In addition, refer to FIG. 1. FIG. 1 is a diagram of absorption spectra of different types of hemoglobin. As shown in FIG. 1, absorption rates of Hb (deoxyhemoglobin) and $HbO_2$ (oxyhemoglobin) in human blood are different in different optical bands. For example, at 700 nm, the absorption rate of Hb (deoxyhemoglobin) is higher than that of $HbO_2$ (oxyhemoglobin), while at 900 nm, the absorption rate of $HbO_2$ (oxyhemoglobin) is higher than that of Hb (deoxyhemoglobin).

When light is converted into an electrical signal, the obtained signal can be divided into an alternating current AC signal and a direct current DC signal because the absorption of light by arteries changes while the absorption of light by other tissues remains basically unchanged. By extracting the AC signal, characteristics of blood flow can be reflected. This technology is referred to as photoplethysmography (PPG).

A PPG module is usually used on a smart wearable device. The PPG module detects human body features such as a heart rate and blood oxygen saturation of a user based on the photoplethysmography PPG technology. The PPG module mainly includes an analog front end (Analog Front End, AFE), a light emitting diode (light emitting diode, LED) light source, and a photodetector (photodetector, PD). A detection principle of the PPG module is: An optical signal is emitted by the LED; after the optical signal is transmitted to skin, inside the skin, a part of the optical signal is absorbed by a human body tissue (including blood), and a part of the optical signal is scattered and reflected; a part of the scattered and reflected optical signal is received by the PD and converted into an electrical signal, where the scattered and reflected optical signal regularly changes as a human pulse beats; and the AFE can detect a pulse wave change status based on a change of the electrical signal detected by the PD, and can further determine data such as the heart rate and blood oxygen based on the pulse wave change status.

Specifically, during operation of the PPG module, the AFE drives the LED to emit optical signals of different wavelengths in time division. After the optical signal is transmitted to the skin, inside the skin, a part of the optical signal is absorbed by the human body tissue (including blood), and the other part of the optical signal is scattered and reflected. A part of the scattered and reflected optical signal is received by the PD and is converted into an electrical signal. The AFE may amplify and process the electrical signal after receiving the electrical signal output by the PD, to obtain PPG signals of different wavelengths.

The blood oxygen saturation (SPO$_2$) in the blood is calculated by using a plurality of PPG signals of different wavelengths (such as red light and infrared light). An empirical formula for calculating the blood oxygen saturation is:

$$SPO_2 = a \times R + b$$

where R=PI(RED)/PI(IR), PI(RED) is a ratio of an alternating current AC signal to a direct current DC signal that are separated from a red-light PPG signal, PI(IR) is a ratio of an alternating current AC signal to a direct current DC signal that are separated from an infrared-light PPG signal, a and b are fitting coefficients, the blood oxygen saturation SPO$_2$ can be measured by a medical pulse oximeter, an R value can be obtained, and a and b are then obtained by fitting according to a data fitting method.

The three components, namely, the PD, the LED, and the AFE, in an existing PPG module each are of an independent structure. Separately disposing of the PD, the LED, and the AFE may cause a large volume of the PPG module, and may occupy a large space of an electronic device. This is not conducive to miniaturization of an electronic device in which the PPG module is used.

Moreover, for an existing solution in which the PD, the LED, and the AFE are integrated into one PPG module, an integration process causes high costs of the PPG module, which is not conducive to reduction of costs of the electronic device using the PPG module. In addition, because the PD of the PPG module can collect optical signals of different wavelengths only in time division, one of the collected red light signal and infrared light signal is delayed compared with the other, that is, the red light signal and the infrared light signal are not obtained simultaneously, tracking of the physiological change data is poor, and accuracy of a calculated blood oxygen saturation value is low.

To resolve the foregoing technical problem, an embodiment of this application provides a test circuit. The test circuit provided in this embodiment of this application is applicable to an electronic device. The electronic device includes, but is not limited to, a smart wearable device (a wearable device that can support human health monitoring, such as a smartwatch, a smart band, or a smart eye mask), a mobile phone, a tablet computer, a notebook computer, a medical device, a smart home appliance, and the like. For ease of understanding, exemplary descriptions are provided below by using an example in which the test circuit is applied to a mobile phone.

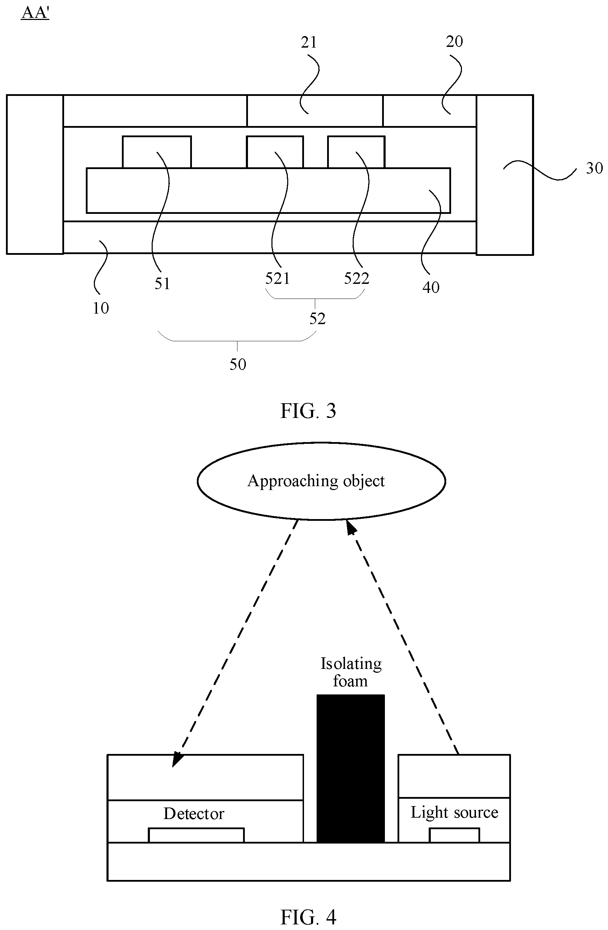

Refer to FIG. 2 and FIG. 3. FIG. 2 is a schematic diagram of a structure of an electronic device according to an embodiment of this application. FIG. 3 is a cross-sectional view of the electronic device shown in FIG. 2 in a direction A-A'. The electronic device 100 includes a display module 10, a rear cover (or referred to as a battery cover) 20, and a middle frame 30.

The display module 10 includes a cover plate and a display screen that are stacked. The cover plate can protect the display screen. The display screen may include a liquid crystal display (Liquid Crystal Display, LCD), an organic light emitting diode (Organic Light Emitting Diode, OLED) display screen, an LED display screen, and the like. The LED display screen may include a Micro-LED display screen, a Mini-LED display screen, and the like. A type of the display screen is not limited in this embodiment of this application.

For example, the display screen 12 includes a display region and a non-display region disposed around the display region. A plurality of subpixels for emitting light is disposed in the display region, and a driving circuit configured to drive the subpixels in the display region to emit light is disposed in the non-display region.

A material of the rear cover 20 may include a non-transparent material such as plastic, vegan leather, and glass fiber; or may include a transparent material such as glass. The material of the rear cover 20 is not limited in this embodiment of this application.

The middle frame 30 includes an annular exterior member 31 and a support member (not shown in the figure) that is located in the annular exterior member 31 and that is located between the display screen 10 and the rear cover 20. The rear cover 20 and the middle frame 30 may be of an integral structure or may be of an independent structure. This is not limited in this embodiment of this application.

The cover plate, the rear cover (also referred to as a battery cover) 20, and the annular exterior member 31 enclose an accommodating cavity. Structures such as a printed circuit board (Printed Circuit Board) 40 and a functional device 50 are disposed in the accommodating cavity. The functional device 50 includes a first functional assembly and a second functional assembly. The first functional assembly may be disposed on the PCB 40 and is electrically connected to the PCB 40. The second functional assembly may not be disposed on the PCB 40 but is electrically connected to the PCB 40. The first functional assembly may include components such as a processor 51, a test circuit 52, and a display driving chip (a chip configured to drive a display screen to perform display). The second functional assembly may include components such as a camera 53 and a flash light 54. The display screen and the flash light 54 are disposed on opposite sides of the mobile phone, and the camera 53 and the flash light 54 are located on a same side of the electronic device. Certainly, the functional device 50 may alternatively include only the first functional assembly; or include only the second functional assembly. The components are electrically connected by using the PCB 40, thereby implementing signal transmission and exchange. The support member may support a part of structures in the accommodating cavity.

The test circuit 52 includes a light-emitting assembly 521, an optical proximity sensor 522, and the like. The test circuit 52 tests a PPG signal, and further determines, based on the PPG signal, human vital sign parameters such as a heart rate, a respiratory rate, blood oxygen, a blood pressure, blood glucose, a perfusion index, a pulse variability index, and content of blood indicators such as carboxyhemoglobin and glycated hemoglobin.

The optical proximity sensor 522 is usually used on an electronic device (such as a mobile phone, a smart wearable device, and a tablet computer), to detect whether there is an object near the electronic device 100 and proximity of the object. For example, the electronic device 100 may detect, by using the optical proximity sensor, that a user holds the electronic device 100 close to an ear for a call, so that automatic screen-off is implemented to achieve power saving. Refer to FIG. 4. FIG. 4 is a diagram of a working principle of an optical proximity sensor. As shown in FIG. 4, the working principle of the optical proximity sensor is that an emitting light source, for example, an infrared light source, emits infrared light. The infrared light source may be an infrared LED, an infrared vertical cavity surface emitting laser (Vertical Cavity Surface Emitting Laser, VCSEL), or the like. In addition, an infrared light detector is used to receive infrared light reflected by an approaching object, and an intensity of the reflected infrared light is integrated, an integral value is defined as proximity data (Proximity data, PDATA), and an approaching distance of an external object may be sensed based on a value of the PDATA. To avoid that a part of infrared light emitted by the infrared light source is reflected by an internal structural part or diffracted to, or directly reaches the infrared light detector from the outside, a black isolating foam may be added between the infrared light detector and the infrared light source, to avoid light cross talk and improve a signal-to-noise ratio.

The optical proximity sensor 522 is widely applied and has a low price. Therefore, the test circuit 52 provided in this embodiment of this application detects the PPG signal by using the optical proximity sensor, which can reduce costs. In addition, because structures for collecting an optical signal and processing the optical signal (specific structures and specific working processes are described below) are integrated inside the optical proximity sensor 522, compared with the PPG module in the related art, the test circuit 52 in this embodiment of this application has a smaller volume.

It may be understood that, to achieve an objective of sensing a proximity distance of an external object, generally, there is only one emitting light source in an existing optical proximity sensor, and an optical signal emitted by the emitting light source is only an optical signal of one wavelength. The emitting light source may be integrated into the optical proximity sensor, or may be independently disposed. Because the human vital sign parameter needs to be determined by using at least two optical signals of different wavelengths, a light-emitting unit may be separately disposed in the test circuit 52 in this embodiment of this application based on the optical proximity sensor 522. When the test circuit 52 needs to emit an optical signal by using an emitting light source in the optical proximity sensor 522 to implement PPG signal detection, the light-emitting unit and the emitting light source in the optical proximity sensor 522 are collectively referred to as the light-emitting assembly 521, and the light-emitting unit and the emitting light source in the optical proximity sensor 522 emit optical signals of at least two different wavelengths. When the test circuit 52 does not need to emit an optical signal by using an emitting light source in the optical proximity sensor 522 to implement PPG signal detection, at least two light-emitting units are needed to form the light-emitting assembly 521, so that the light-emitting assembly 521 emits optical signals of at least two different wavelengths.

It may be understood that FIG. 2, FIG. 3, and the related figures below only schematically show some of the components included in the electronic device 100. In practice, the electronic device 100 may have more or fewer components than those shown in the figures, or some components may be combined, or some components may be split, or a different component arrangement may be used. The parts shown in the figures may be implemented by hardware, software, or a combination of software and hardware.

It should be noted that in FIG. 2, the mobile phone 100 is in a shape of a rectangular flat plate. In other optional embodiments, the electronic device may alternatively be in a shape of a square tablet, a circular tablet, an elliptical tablet, or the like. Certainly, the electronic device may alternatively be a foldable electronic device, or the like.

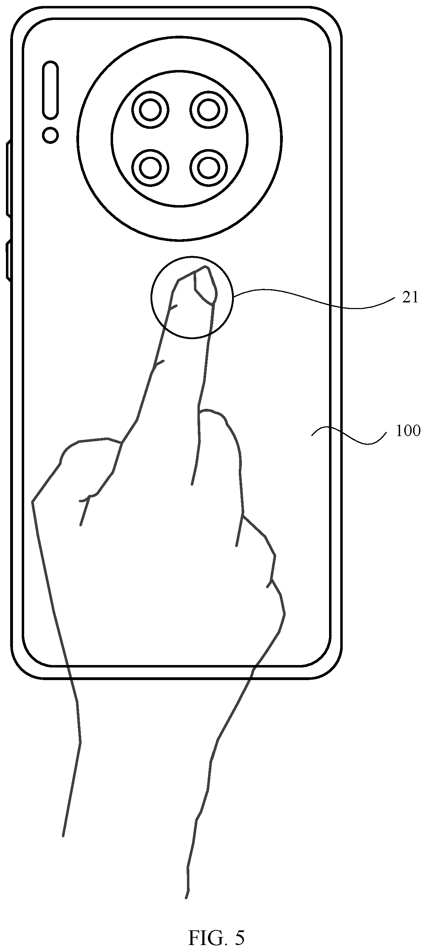

Still refer to FIG. 2 and FIG. 3. The rear cover 20 includes a light-transmissive portion 21, and an optical signal emitted by the light-emitting assembly 521 may emitted out through the light-transmissive portion 21. Refer to FIG. 5. FIG. 5 is a diagram of an application scenario of an electronic device according to an embodiment of this application. When the user touches a light-transmissive portion 21 by using a fingertip, an optical signal emitted by the light-emitting assembly 521 is emitted out through the light-transmissive portion 21, and then illuminates the fingertip of the user. The optical signal passes through a skin tissue of the fingertip, and is then reflected to the optical proximity sensor 522. The optical proximity sensor 522 converts the light into an electrical signal, and determines a PPG signal based on the electrical signal.

A specific structure and a working principle of the test circuit provided in this embodiment of this application are described in detail below. The specific structure and the working principle of the test circuit provided in this embodiment of this application are described in detail by using a case in which an optical proximity sensor cooperates with a light-emitting assembly to implement a PPG test function in time division and a case in which an optical proximity sensor cooperates with a light-emitting assembly to implement a PPG test function at the same time.

First, the specific structure and the working principle of the test circuit provided in this embodiment of this application are described in detail by using the case in which the optical proximity sensor cooperates with the light-emitting assembly to implement the PPG test function in time division.

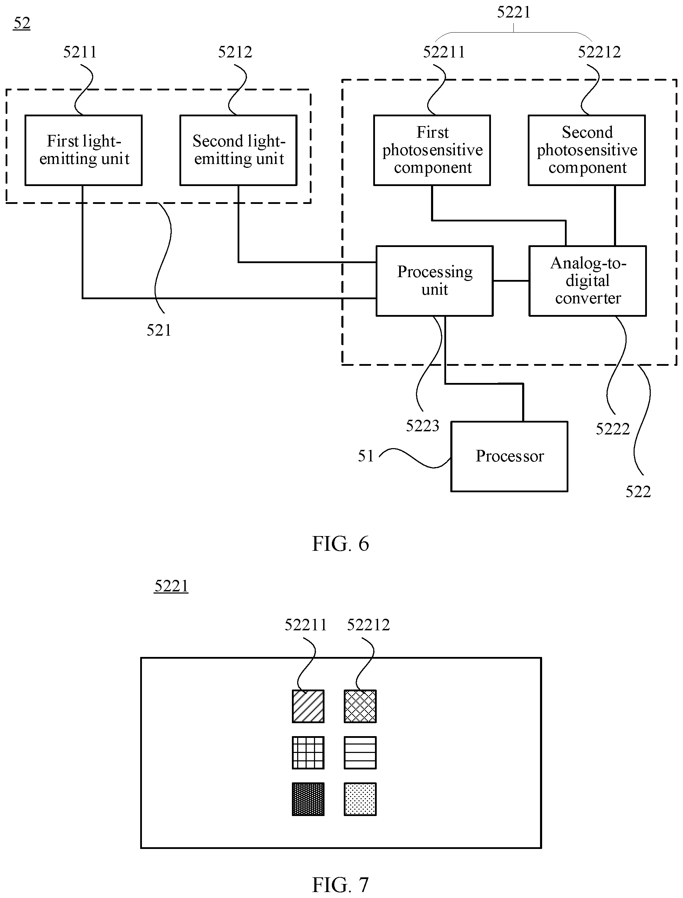

Refer to FIG. 6. FIG. 6 is a circuit diagram of a test circuit according to an embodiment of this application. As shown in FIG. 6, the test circuit 52 includes: the light-emitting assembly 521 and the optical proximity sensor 522. The light-emitting assembly 521 includes at least a first light-emitting unit 5211 and a second-light-emitting unit 5212. The first light-emitting unit 5211 and the second light-emitting unit 5212 both are, for example, components that can emit light, such as an LED light. The first light-emitting unit 5211 may emit a first optical signal, and the second light-emitting unit 5212 may emit a second optical signal. The first optical signal may be a red light signal, and the second optical signal may be an infrared light signal; or the first optical signal may be an infrared light signal, and the second optical signal may be a green light signal. A wavelength range of red light may be 610.0 nm to 699.9.0 nm, a wavelength range of infrared light may be 700.0 nm to 1100.0 nm, and a wavelength range of green light may be 510.0 nm to 549.9 nm. For example, the first optical signal is a red light signal, and a wavelength of the red light signal is 690 nm; and the second optical signal is an infrared light signal, and a wavelength of the infrared light signal is 940 nm. With reference to FIG. 1. When the wavelength of the red light signal is 690 nm and the wavelength of the infrared light signal is 940 nm, absorption rates of Hb (deoxyhemoglobin) and $HbO_2$ (oxyhemoglobin) in human blood are different at 690 nm and 940 nm, and a difference in absorption rate is the greatest. This is conducive to calculation of blood oxygen saturation in blood.

It should be noted that the wavelength range of the red light, the wavelength range of the infrared light, and the wavelength range of the green light are merely examples and do not constitute a limitation on this application.

Refer to FIG. 7. FIG. 7 is a top view of a structure of an optical proximity sensor according to an embodiment of this application. As shown in FIG. 6 and FIG. 7, the optical proximity sensor 522 includes a collection unit 5221 and an analog-to-digital converter 5222. The collection unit 5221 includes a plurality of photosensitive components. FIG. 7 is described by using an example in which the collection unit 5221 includes six photosensitive components. For example, the photosensitive component may include a component that can convert light into an electrical signal, such as a photodetector. The plurality of photosensitive components include at least a first photosensitive component 52211 and a second photosensitive component 52212. Different photosensitive components correspond to optical signals in different bands, that is, the different photosensitive components respond to optical signals in different bands (convert the optical signals into electrical signals). In FIG. 7, patterns filled with different designs represent different photosensitive components, to reflect that the different photosensitive components respond to optical signals in different bands. For example, the first photosensitive component 52211 responds to the first optical signal, and the second photosensitive component 52212 responds to the second optical signal. The first photosensitive component 52211 and the second photosensitive component 52212 are both electrically connected to the analog-to-digital converter 5222. The first photosensitive component 52211 and the analog-to-digital converter 5222, and the second photosensitive component 52212 and the analog-to-digital converter 5222 form two collection channels. The two collection channels are, for example, a first collection channel and a second collection channel.

Optionally, the optical proximity sensor 522 includes a processing unit 5223, the processing unit 5223 is separately electrically connected to the analog-to-digital converter 5222, the first light-emitting unit 5211, and the second light-emitting unit 5212, and the processing unit 5223 is configured to send a drive signal to the first light-emitting unit 5211 and the second light-emitting unit 5212 to drive the first light-emitting unit 5211 and the second light-emitting unit 5212 to emit light. The processing unit 5223 is further configured to process a digital signal output by the analog-to-digital converter 5222. Certainly, a processing unit having a driving capability in the electronic device 100 such as the processor 51 may alternatively drive the first light-emitting unit 5211 and the second light-emitting unit 5212 to emit light, and process a digital signal output by the analog-to-digital converter 5222. This is not limited in this embodiment of this application. In this way, a structure for driving the first light-emitting unit 5211 and the second light-emitting unit 5212 to emit light and a structure for processing data do not need to be separately disposed.

It should be noted that this embodiment of this application is described by using an example in which a processing unit 5223 is disposed in the optical proximity sensor 522, the processing unit 5223 drives the first light-emitting unit 5211 and the second light-emitting unit 5212 to emit light, the first optical signal emitted by the first light-emitting unit 5211 is a red light signal, and the second optical signal emitted by the second light-emitting unit 5212 is an infrared light signal.

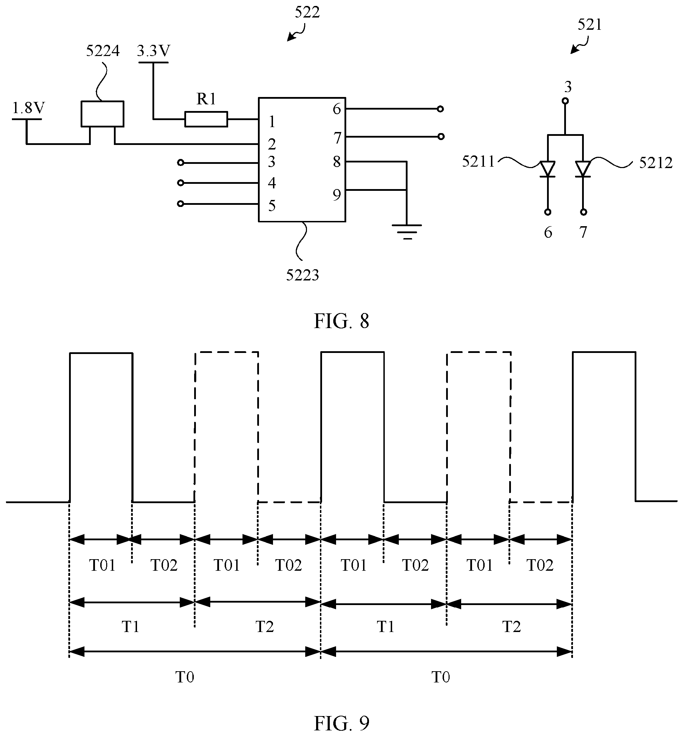

Refer to FIG. 8. FIG. 8 is an example of a test circuit according to an embodiment of this application. As shown in FIG. 8, the test circuit 52 includes the light-emitting assembly 521 and the optical proximity sensor 522. The light-emitting assembly 521 includes the first light-emitting unit 5211 and the second light-emitting unit 5212. The first light-emitting unit 5211 is an external light LED, and the second light-emitting unit 5212 is an infrared light LED. The optical proximity sensor 522 includes the processing unit 5223, a switch 5224, a first resistor R1, the collection unit (not shown in FIG. 7), and the analog-to-digital converter (not shown in FIG. 7). The processing unit 5223 includes a first pin 1, a second pin 2, a third pin 3, a fourth pin 4, a fifth pin 5, a sixth pin 6, a seventh pin 7, an eighth pin 8, and a ninth pin 9. The third pin 3 is electrically connected to a positive terminal of the infrared light LED and a positive terminal of the red light LED. The sixth pin 6 and the seventh pin 7 are respectively electrically connected to a negative terminal of the infrared light LED and a negative terminal of the red light LED. The first pin 1 may receive a voltage of 3.3 V, to supply power to the infrared light LED and the red light LED through the third pin 3. The fourth pin 4 and the fifth pin 5 are I2C communication pins. The eighth pin 8 and the ninth pin 9 are both connected to ground. The second pin 2 is electrically connected to the switch 5224. When the switch 5224 is turned on, a voltage of 1.8 V is transmitted to the second pin 2 through the switch 5224 to supply power to the processing unit 5223, thereby ensuring normal operation of the processing unit 5223. In some embodiments, the first resistor R1 is disposed on the first pin 1, and the first resistor R1 may be coupled to another component to implement a function such as filtering.

It should be noted that FIG. 8 is merely an example of the light-emitting assembly and the optical proximity sensor, and does not constitute a limitation on this application. A person skilled in the art may select the optical proximity sensor and the light-emitting assembly according to an actual situation.

Refer to FIG. 9. FIG. 9 is a sequence diagram of time-division driving of a test circuit according to an embodiment of this application. As shown in FIG. 9, the test circuit 52 includes a plurality of duty cycles T0. Each duty cycle T0 includes at least a first stage T1 and a second stage T2. The first stage T1 corresponds to a collection and processing process of the first optical signal, and the second stage T2 corresponds to a collection and processing process of the second optical signal. In FIG. 9, a solid line represents a collection and processing sequence of the first optical signal, and a dashed line represents a collection and processing sequence of the second optical signal. The first stage T1 and the second stage T2 both include at least one pulse cycle, and the pulse cycle includes a light-emitting stage T01 at which working is performed in a first level (for example, a high level) state and a non-light-emitting stage T02 at which working is performed in a second level (for example, a low level) state. Because the first photosensitive component 52211 and the second photosensitive component 52212 share the analog-to-digital converter 5222, the first collection channel formed by the first photosensitive component 52211 and the analog-to-digital converter 5222 and the second collection channel formed by the second photosensitive component 52212 and the analog-to-digital converter 5222 need to collect and process, in time division (that is, in two different time periods, namely, at the first stage T1 and the second stage T2), the first optical signal emitted by the first light-emitting unit 5211 and the second optical signal emitted by the second light-emitting unit 5212, to distinguish between the first optical signal and the second optical signal.

A specific test method is: At the light-emitting stage T01 of the first stage T1, the processing unit 5223 emits the first drive signal to the first light-emitting unit 5211; the first light-emitting unit 5211 emits a red light signal (also referred to as the first optical signal) based on the first drive signal; after being emitted out through the light-transmissive portion 21, the red light signal is reflected and/or scattered when the red light signal illuminates the skin tissue of the fingertip of the user; and the first photosensitive component 52211 collects ambient light and reflected and/or scattered light of the red light signal (which are collectively referred to as a third optical signal), and converts the optical signal into an electrical signal. The electrical signal is an analog signal. The analog-to-digital converter 5222 converts the analog signal into a digital signal, and the processing unit 5223 processes the digital signal, for example, performs processing such as calibration, filtering, or integration (accumulation of signal intensities within a time period), to obtain an optical signal integral value D1. In other words, the first collection channel formed by the first photosensitive component 52211 and the analog-to-digital converter 5222 collects the ambient light and the reflected and/or scattered light of the red light signal and converts the optical signal into the digital signal, and the processing unit 5223 processes the digital signal, to obtain the integral value D1. At the non-light-emitting stage T02 of the first stage T1, the processing unit 5223 emits a second drive signal to the first light-emitting unit 5211. The first light-emitting unit 5211 does not emit light. The first photosensitive component 52211 collects only the ambient light (also referred to as a fourth optical signal) and converts the optical signal into an electrical signal. The electrical signal is an analog signal. The analog-to-digital converter 5222 converts the analog signal into a digital signal, and the processing unit 5223 processes the digital signal, for example, performs processing such as calibration, filtering, or integration (accumulation of signal intensities within a time period), to obtain an optical signal integral value D2. In other words, the first collection channel formed by the first photosensitive component 52211 and the analog-to-digital converter 5222 collects only the ambient light and converts the optical signal into the digital signal, and the processing unit 5223 processes the digital signal, to obtain the integral value D2. After one pulse cycle of the first stage T1 ends, the processing unit 5223 calculates a difference between the integral value D1 and the integral value D2, to obtain an integral value (including only a first optical signal component part) of a light intensity of a first optical reference signal, to exclude interference from an ambient light direct current component. The integral value of the light intensity of the first optical reference signal is then sent to the processor 51.

It should be noted that reflection and/or scattering in this embodiment of this application may be only reflection, only scattering, or both reflection and scattering.

At the light-emitting stage T01 of the second stage T2, the processing unit 5223 emits a first drive signal to the second light-emitting unit 5212; the second light-emitting unit 5212 emits an infrared light signal (also referred to as the second optical signal) based on the first drive signal; after being emitted out through the light-transmissive portion 21, the infrared light signal is reflected and/or scattered when the infrared light signal illuminates the skin tissue of the fingertip of the user; and the second photosensitive component 52212 collects the ambient light and reflected and/or scattered light of the infrared light signal (which are collectively referred to as a fifth optical signal) and converts the optical signal into an electrical signal. The electrical signal is an analog signal. The analog-to-digital converter 5222 converts the analog signal into a digital signal, and the processing unit 5223 processes the digital signal, for example, performs processing such as calibration, filtering, or integration (accumulation of signal intensities within a time period), to obtain an optical signal integral value D3. In other words, the second collection channel formed by the second photosensitive component 52212 and the analog-to-digital converter 5222 collects the ambient light and reflected and/or scattered light of the infrared light signal and converts the optical signal into the digital signal, and the processing unit 5223 processes the digital signal, to obtain the integral value D3. At the non-light-emitting stage T02 of the second stage T2, the processing unit 5223 emits a second drive signal to the second light-emitting unit 5212. The second light-emitting unit 5212 does not emit light. The second photosensitive component 52212 collects only the ambient light (also referred to as a sixth optical signal) and converts the optical signal into an electrical signal. The electrical signal is an analog signal. The analog-to-digital converter 5222 converts the analog signal into a digital signal, and the processing unit 5223 processes the digital signal, for example, performs processing such as calibration, filtering, or integration (accumulation of signal intensities within a time period), to obtain an optical signal integral value D4. In other words, the second collection channel formed by the second photosensitive component 52212 and the analog-to-digital converter 5222 collects only the ambient light and converts the optical signal into the digital signal, and the processing unit 5223 processes the digital signal, to obtain the integral value D4. After one pulse cycle of the second stage T2 ends, the optical proximity sensor 522 calculates a difference between the integral value D3 and the integral value D4, to obtain an integral value (including only a second optical signal component part) of a light intensity of a second optical reference signal, to exclude interference from an ambient light direct current component. The integral value of the light intensity of the second optical reference signal is then sent to the processor 51.

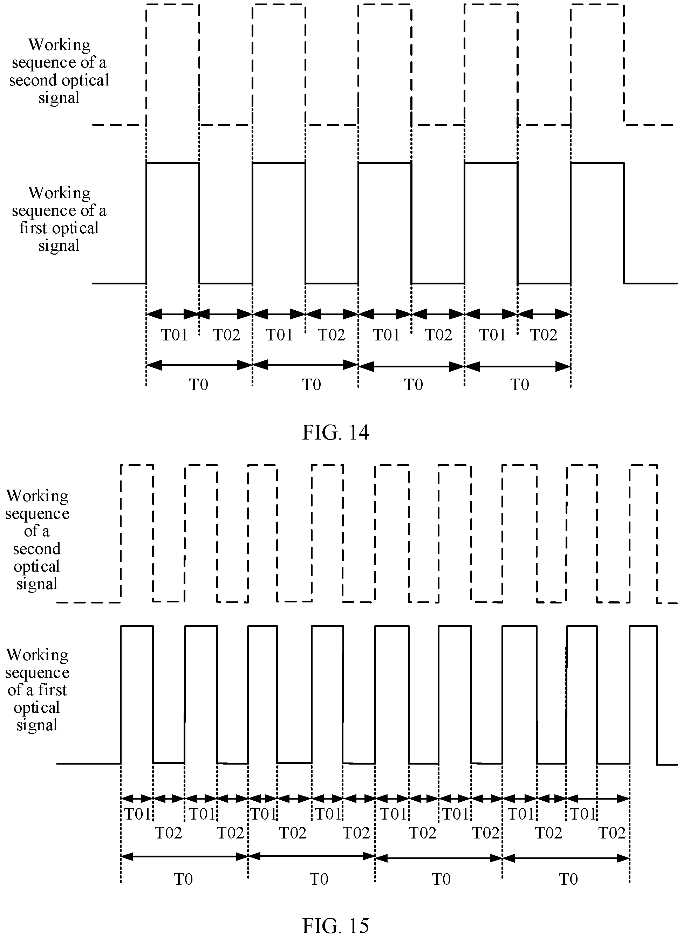

Because the test circuit includes a plurality of duty cycles T0, in the foregoing manner, the processor 51 obtains the integral value of the light intensity of the first optical reference signal within a time period. That is, the plurality of duty cycles T0 within the time period correspond to integral values of light intensities of a plurality of first optical reference signals. The integral values of the light intensities of the plurality of first optical reference signals may form a curve, and the curve represents an intensity of the red light signal (that is, a red light PPG signal), as pointed by an arrow ② shown in FIG. 15. FIG. 15 is described in detail below, and details are not described herein. Moreover, the processor 51 obtains the integral value of the light intensity of the second optical reference signal within a time period. That is, the plurality of duty cycles T0 within the time period correspond to integral values of light intensities of the plurality of second optical reference signals. The integral values of the light intensities of the plurality of second optical reference signals may form a curve, and the curve represents an intensity of the infrared light signal (that is, an infrared light PPG signal), as pointed by an arrow ① shown in FIG. 15. The processor 51 may separate an alternating current AC signal and a direct current DC signal of the red light and separate an alternating current AC signal and a direct current DC signal of the infrared light based on the red light PPG signal and the infrared light PPG signal, to obtain a ratio PI(RED) of the alternating current AC signal to the direct current DC signal of the red light and a ratio PI(IR) of the alternating current AC signal to the direct current DC signal of the infrared light, determine an R value based on PI(RED) and PI(IR), and further determine human body features such as the heart rate and the blood oxygen saturation.

It should be noted herein that the test circuit not only can calculate the blood oxygen saturation, the heart rate, and the like based on signals in two different optical bands (for example, the first optical signal and the second optical signal), but also can couple optical signals in other bands, for example, determine a PPG signal based on optical signals in three (where the collection unit 5221 correspondingly includes three photosensitive components), four (where the collection unit 5221 correspondingly includes four photosensitive components), five (where the collection unit 5221 correspondingly includes five photosensitive components), or six (where the collection unit 5221 correspondingly includes six photosensitive components) optical bands. to calculate, based on the PPG signal, the human vital sign parameters such as a blood pressure, blood glucose, a perfusion index, a pulse variability index, and content of blood indicators such as carboxyhemoglobin and glycated hemoglobin.

During actual application, there may be another flicker light source outside the electronic device 100. Because the flicker light source is a light source that is switched between bright and dark states at a particular frequency, the difference between the integral value D1 and the integral value D2 may be different from an actual difference, resulting in integral fluctuation (occurrence of a maximum value or a minimum value) of the light intensity of the first optical reference signal, and even resulting in a failure in a function of the optical proximity sensor 522. Alternatively, the difference between the integral value D3 and the integral value D4 is different from an actual difference, resulting in integral fluctuation (occurrence of a maximum value or a minimum value) of the light intensity of the second optical reference signal, and even resulting in a failure in a function of the optical proximity sensor 522. Alternatively, the difference between the integral value D1 and the integral value D2 is different from an actual difference, and the difference between the integral value D3 and the integral value D4 is different from an actual difference, resulting in fluctuation (occurrence of a maximum value and a minimum value) in both the integral value of the light intensity of the first optical reference signal and the integral value of the light intensity of the second optical reference signal, and even resulting in a failure in a function of the optical proximity sensor 522.

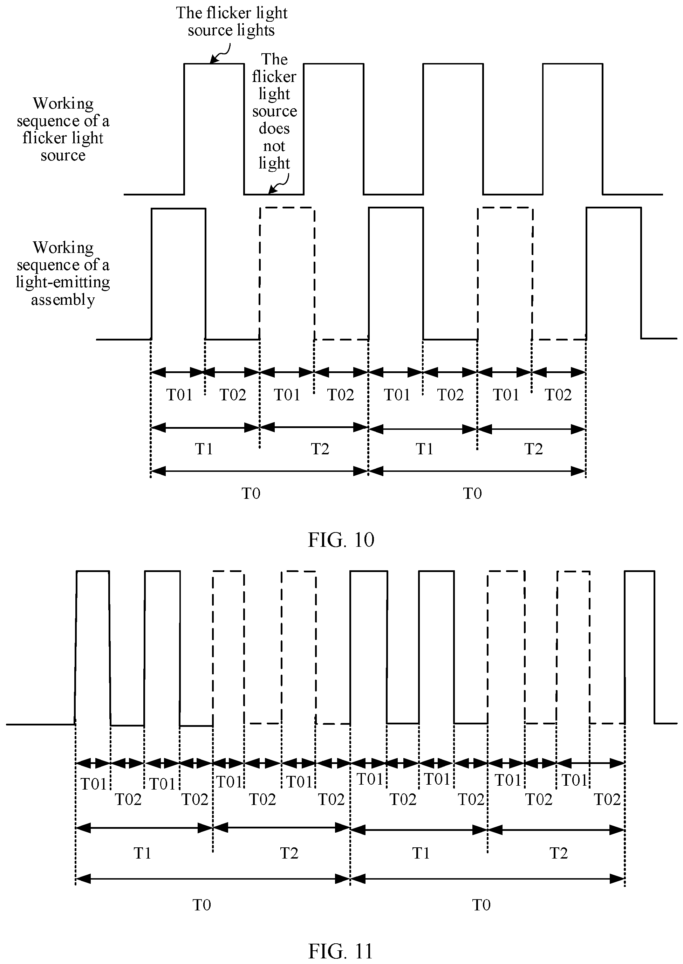

For example, refer to FIG. 10. FIG. 10 is a diagram of comparison between a working sequence of a light-emitting assembly and a working sequence of a flicker light source according to an embodiment of this application. As shown in FIG. 10, at the light-emitting stage T01 of the first stage T1, the first collection channel formed by the first photosensitive component 52211 and the analog-to-digital converter 5222 still collects, for example, the ambient light and the reflected and/or scattered light of the red light signal (which are collectively referred to as the third optical signal), and therefore, the integral value D1 remains unchanged. At the non-light-emitting stage T02 of the first stage T1, the first collection channel formed by the first photosensitive component 52211 and the analog-to-digital converter 5222 collects the ambient light and the external flicker light source. Therefore, the integral value D2 becomes larger. After one pulse cycle of the first stage T1 ends, the difference that is between the integral value D1 and the integral value D2 and that is calculated by the processing unit 5223 is less than an actual value.

To isolate interference from the external flicker light source, refer to FIG. 11. FIG. 11 is another sequence diagram of time-division driving of a test circuit according to an embodiment of this application. In FIG. 11, a solid line represents a sequence of collection and processing of the first optical signal, and a dashed line represents a sequence of collection and processing of the second optical signal. As shown in FIG. 11, the first stage T1 and the second stage T2 of each duty cycle T0 both include N pulse cycles, where N≥2, and N is a positive integer. In other words, a single pulse cycle in FIG. 9 is divided into several pulse cycles. FIG. 11 is described by using an example in which the first stage T1 and the second stage T2 of each duty cycle T0 both include two pulse cycles.

This is because, when duration of a pulse cycle is long, an external flicker light source may affect each pulse cycle, that is, an integral value of a light intensity of each first optical reference signal is different from an actual value; or an integral value of a light intensity of each second optical reference signal is different from an actual value; or an integral value of a light intensity of each first optical reference signal and an integral value of a light intensity of each second optical reference signal are both different from actual values, and consequently, a PPG test result is affected. When both the first stage T1 and the second stage T2 of each duty cycle T0 include the N pulse cycles, the external flicker light source may affect only a part of the pulse cycles. For example, when one or more pulse cycles (a pulse cycle after pulse duration is shortened) are located in a dark region of the external flicker light source, the one or more pulse cycles are not affected, that is, only integral values of light intensities of a part of first optical reference signals are affected; or only integral values of light intensities of a part of second optical reference signals are affected; or integral values of light intensities of a part of first optical reference signals and integral values of light intensities of a part of second optical reference signals are affected, thereby reducing an impact of the flicker light source and improving anti-interference capability.

The foregoing describes in detail that the optical proximity sensor cooperates with the light-emitting assembly to implement a PPG test function in time division. It can be learned from the foregoing content that, because the optical proximity sensor is commonly used in an electronic device, and has a large supply volume, low costs, and a small volume, replacing the existing PPG module with the light-emitting assembly and the optical proximity sensor to implement the PPG detection can reduce costs of a test circuit and a volume occupied in the electronic device. In addition, both the first stage T1 and the second stage T2 of each duty cycle T0 of the test circuit are set to include a plurality of pulse cycles, thereby improving bandpass matching and ensuring a better anti-interference feature.

The specific structure and the working principle of the test circuit provided in this embodiment of this application are described below in detail by using the case in which the optical proximity sensor cooperates with the light-emitting assembly to implement the PPG test function at the same time.

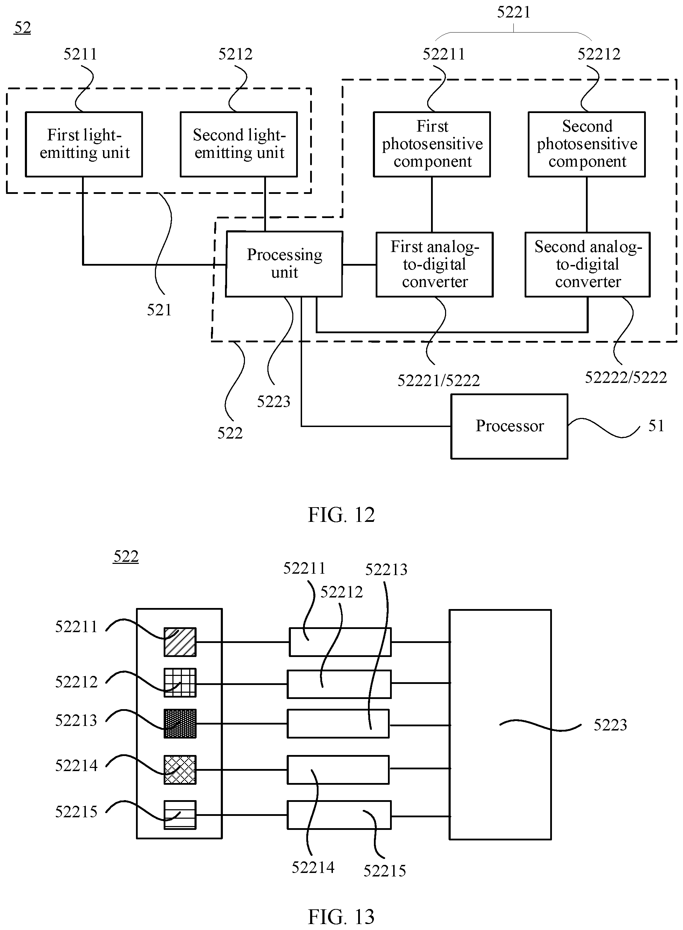

Refer to FIG. 12. FIG. 12 is a circuit diagram of another test circuit according to an embodiment of this application. As shown in FIG. 12, different from the foregoing, the optical proximity sensor 522 includes at least two analog-to-digital converters 5222, the at least two analog-to-digital converters 5222 include a first analog-to-digital converter 52221 and a second analog-to-digital converter 52222, the first photosensitive component 52211 is electrically connected to the first analog-to-digital converter 52221, and the second photosensitive component 52212 is electrically connected to the second analog-to-digital converter 52222. The first photosensitive component 52211 and the first analog-to-digital converter 52221, and the second photosensitive component 52212 and the second analog-to-digital converter 52222 form two collection channels. The two collection channels are, for example, a first collection channel and a second collection channel.

For example, refer to FIG. 13. FIG. 13 is an example of an optical proximity sensor according to an embodiment of this application. As shown in FIG. 13, the optical proximity sensor 522 includes the processing unit 5223, the collection unit 5221, and five analog-to-digital converters 5222. The collection unit 5221 includes the first photosensitive component 52211, the second photosensitive component 52212, the third photosensitive component 52213, a fourth photosensitive component 52214, and a fifth photosensitive component 52215. The first photosensitive component 52211 may convert an infrared light signal into an electrical signal. The second photosensitive component 52212 may convert a red light signal into an electrical signal. The third photosensitive component 52213 may convert a green light signal into an electrical signal. The fourth photosensitive component 52214 may convert a blue light signal into an electrical signal. The fifth photosensitive component 52215 may convert a purple light signal into an electrical signal. In FIG. 13, patterns filled with different designs represent different photosensitive components, to reflect that the different photosensitive components respond to optical signals in different bands. The five analog-to-digital converters include the first analog-to-digital converter 52221, the second analog-to-digital converter 52222, the third analog-to-digital converter 52223, the fourth analog-to-digital converter 52224, and the fifth analog-to-digital converter 52225. The first analog-to-digital converter 52221 is electrically connected to the first photosensitive component 52211, to convert an electrical signal converted by the first photosensitive component 52211 into a digital signal. The second analog-to-digital converter 52222 is electrically connected to the second photosensitive component 52212, to convert an electrical signal converted by the second photosensitive component 52212 into a digital signal. The third analog-to-digital converter 52223 is electrically connected to the third photosensitive component 52213, to convert an electrical signal converted by the third photosensitive component 52213 into a digital signal. The fourth analog-to-digital converter 52224 is electrically connected to the fourth photosensitive component 52214, to convert an electrical signal converted by the fourth photosensitive component 52214 into a digital signal. The fifth analog-to-digital converter 52225 is electrically connected to the fifth photosensitive component 52215, to convert the electrical signal converted by the fifth photosensitive component 52215 into a digital signal.

The following is described by using an example in which the optical proximity sensor 522 includes two photosensitive components and two analog-to-digital converters 5222. Refer to FIG. 14. FIG. 14 is a sequence diagram of simultaneous driving of a test circuit according to an embodiment of this application. As shown in FIG. 14, the test circuit 52 includes the plurality of duty cycles T0. Each duty cycle T0 includes at least one pulse cycle. The pulse cycle includes the light-emitting stage T01 working in the first level (for example, a high level) state and the non-light-emitting stage T02 working in the second level (for example, a low level) state. In FIG. 13, a solid line represents a sequence of collection and processing of the first optical signal, and a dashed line represents a sequence of collection and processing of the second optical signal. Because the first photosensitive component 52211 and the second photosensitive component 52212 respectively correspond to different analog-to-digital converters 5222, the first collection channel formed by the first photosensitive component 52211 and the analog-to-digital converter 5222 and the second collection channel formed by the second photosensitive component 52212 and the analog-to-digital converter 5222 may collect and process the first optical signal emitted by the first light-emitting unit 5211 and the second optical signal emitted by the second light-emitting unit 5212 at the same time (that is, at the light-emitting stage T01). That is, different collection channels may collect optical signals in different bands at the same time, to collect and process optical signals in different bands at the same time.

A specific test method is as follows: At the light-emitting stage T01, the processing unit 5223 separately emits a first drive signal to the first light-emitting unit 5211 and the second light-emitting unit 5212; the first light-emitting unit 5211 emits a red light signal (also referred to as the first optical signal) based on the first drive signal; the second light-emitting unit 5212 emits an infrared light signal (also referred to as the second optical signal) based on the first drive signal; after being emitted through the light-transmissive portion 21, the red light signal and the infrared light signal are reflected and/or scattered when illuminating on the skin tissue of the fingertip of the user; and the first photosensitive component 52211 collects ambient light and reflected and/or scattered light of the red light signal (collectively referred to as a seventh optical signal) and converts the optical signal into an electrical signal. The electrical signal is an analog signal. The first analog-to-digital converter 52221 converts the analog signal into a digital signal, and the processing unit 5223 processes the digital signal, for example, performs processing such as calibration, filtering, or integration (accumulation of signal intensity within a time period), to obtain an optical signal integral value D5. The second photosensitive component 52212 collects the ambient light and reflected and/or scattered light of the infrared light signal (collectively referred to as an eighth optical signal) and converts the optical signal into an electrical signal. The electrical signal is an analog signal. The second analog-to-digital converter 52222 converts the analog signal into a digital signal, and the processing unit 5223 processes the digital signal, for example, performs processing such as calibration, filtering, or integration (accumulation of signal intensity within a time period), to obtain an optical signal integral value D6. In other words, the first collection channel formed by the first photosensitive component 52211 and the first analog-to-digital converter 52221 collects the ambient light and the reflected and/or scattered light of the red light signal and converts the optical signal into the digital signal, and the processing unit 5223 processes the digital signal, to obtain the integral value D5. At the same time, the second collection channel formed by the second photosensitive component 52212 and the second analog-to-digital converter 52222 collects the ambient light and reflected and/or scattered light of the infrared light signal, converts the optical signal into a digital signal, and the processing unit 5223 processes the digital signal, to obtain the integral value D6.

At the non-light-emitting stage T02, the processing unit 5223 separately emits a second drive signal to the first light-emitting unit 5211 and the second light-emitting unit 5212, the first light-emitting unit 5211 and the second light-emitting unit 5212 do not emit light, and the first photosensitive component 52211 collects only ambient light (also referred to as a ninth optical signal) and converts the optical signal into an electrical signal. The electrical signal is an analog signal. The first analog-to-digital converter 52221 converts the analog signal into a digital signal, and the processing unit 5223 processes the digital signal, for example, performs processing such as calibration, filtering, or integration (accumulation of signal intensity within a time period), to obtain an optical signal integral value D7. The second photosensitive component 52212 collects only the ambient light (also referred to as a tenth optical signal) and converts the optical signal into an electrical signal. The electrical signal is an analog signal. The second analog-to-digital converter 52222 converts the analog signal into a digital signal, and the processing unit 5223 processes the digital signal, for example, performs processing such as calibration, filtering, or integration (accumulation of signal intensity within a time period), to obtain an optical signal integral value D8. In other words, the first collection channel formed by the first photosensitive component 52211 and the first analog-to-digital converter 52221 collects only the ambient light and converts the optical signal into the digital signal, and the processing unit 5223 processes the digital signal, to obtain the integral value D7. At the same time, the second collection channel formed by the second photosensitive component 52212 and the second analog-to-digital converter 52222 collects only the ambient light and converts the optical signal into a digital signal, and the processing unit 5223 processes the digital signal to obtain the integral value D8. After one pulse cycle ends, the processing unit 5223 calculates a difference between the integral value D5 and the integral value D7, to obtain an integral value (including only the first optical signal component part) of the light intensity of the first optical reference signal, and calculates a difference between the integral value D6 and the integral value D8, to obtain an integral value (including only the second optical signal component part) of the light intensity of the second optical reference signal, to exclude interference of the ambient light direct current component. The integral value of the light intensity of the first optical reference signal and the integral value of the light intensity of the second optical reference signal are then sent to the processor 51.

Because the test circuit includes a plurality of duty cycles T0, in the foregoing manner, the processor 51 obtains the integral value of the light intensity of the first optical reference signal within a time period. That is, the plurality of duty cycles T0 within the time period correspond to integral values of light intensities of a plurality of first optical reference signals. The integral values of the light intensities of the plurality of first optical reference signals may form a curve, and the curve represents an intensity of the red light signal (that is, a red light PPG signal). Moreover, the processor 51 obtains the integral value of the light intensity of the second optical reference signal within a time period. That is, the plurality of duty cycles T0 within the time period correspond to integral values of light intensities of the plurality of second optical reference signals. The integral values of the light intensities of the plurality of second optical reference signals may form a curve, and the curve represents an intensity of the infrared light signal (that is, an infrared light PPG signal). The processor 51 may separate an alternating current AC signal and a direct current DC signal of the red light and separate an alternating current AC signal and a direct current DC signal of the infrared light based on the red light PPG signal and the infrared light PPG signal, to obtain a ratio PI(RED) of the alternating current AC signal to the direct current DC signal of the red light and a ratio PI(IR) of the alternating current AC signal to the direct current DC signal of the infrared light, determine an R value based on PI(RED) and PI(IR), and further determine human body features such as the heart rate and the blood oxygen saturation.

It should be noted herein that, the test circuit can calculate the blood oxygen saturation, the heart rate, and the like based on signals in two different optical bands (for example, the first optical signal and the second optical signal), and can further couple optical signals in other bands, for example, determine a PPG signal based on optical signals in three (correspondingly, the collection unit 5221 includes three photosensitive components, and there are at least three analog-to-digital converters 5222), four (correspondingly, the collection unit 5221 includes four photosensitive components, and there are at least four analog-to-digital converters 5222), five (correspondingly, the collection unit 5221 includes five photosensitive components, and there are at least five analog-to-digital converters 5222), or six (correspondingly, the collection unit 5221 includes six photosensitive components, and there are at least six analog-to-digital converters 5222) optical bands. to calculate, based on the PPG signal, the human vital sign parameters such as a blood pressure, blood glucose, a perfusion index, a pulse variability index, and content of blood indicators such as carboxyhemoglobin and glycated hemoglobin.

Similarly, to isolate interference from the external flicker light source, refer to FIG. 15. FIG. 15 is another sequence diagram of simultaneous driving of a test circuit according to an embodiment of this application. In FIG. 15, a solid line represents a sequence of collection and processing of the first optical signal, and a dashed line represents a sequence of collection and processing of the second optical signal. As shown in FIG. 15, each duty cycle T0 includes N pulse cycles, where N≥2, and N is a positive integer. In other words, a single pulse cycle in FIG. 14 is divided into several pulse cycles. FIG. 15 is described by using an example in which each duty cycle T0 includes two pulse cycles. A specific principle of reducing an impact of a flicker light source and improving an anti-interference capability is similar to the foregoing content by including N pulse cycles in each duty cycle T0. For details, refer to the foregoing content, and details are not described herein again.

The foregoing describes in detail that the optical proximity sensor cooperates with the light-emitting assembly to simultaneously implement the PPG test function. It can be learned from the foregoing that, the existing PPG module is replaced with the light-emitting assembly and the optical proximity sensor to implement a PPG test, the plurality of analog-to-digital converters are disposed in the optical proximity sensor, the plurality of photosensitive components and the plurality of analog-to-digital converters form different collection channels in a one-to-one correspondence, and the different collection channels can simultaneously collect and process optical signals in different bands. The integral value of the light intensity of the first optical reference signal and the integral value of the light intensity of the second optical reference signal that are measured at a same time point are compared with the optical signals in different wave bands that are obtained in time division (that is, at different moments). Because the vital sign parameters are determined based on the optical signals in different bands, the optical signals in different bands obtained in time division cause a time difference between vital signs of the user, which cannot better reflect real vital signs. In this solution, optical signals in different bands at a same moment can be obtained, that is, the vital signs of the user at the same moment can be reflected, which has tracking on the physiological change data of the user, and can more truly reflect the vital signs. Based on this, longer duration can be reserved for the analog-to-digital converter to perform optical signal processing, so that integration detection duration can be improved, and better sensitivity can be achieved.

In this case (collecting and processing optical signals in different bands at the same time), a broad-spectrum light source may be used for the light-emitting assembly, and the broad-spectrum light source is a light source emitting an optical signal formed by optical signals of at least two different wavelengths. The wide-spectrum light source has a relatively wide wavelength range, for example, from 380 nm to 780 nm. For example, the wide-spectrum light source emits, for example, white light. An optical signal emitted by the wide-spectrum light source includes light of seven colors: red, orange, yellow, green, blue, indigo, and purple. Red light may be used as the first optical signal, and green light may be used as the second optical signal. That is, when the wide-spectrum light source emits white light, the first collection channel may collect and process only red light in the white light, and the second collection channel may collect and process only green light in the white light.

When a broad-spectrum light source is selected for the light-emitting assembly, different light-emitting units do not need to be disposed, and only one light source needs to be disposed, thereby reducing costs of the test circuit, and reducing an area occupied by the PCB 40 in the electronic device.

Further, it is considered that the light emitted by the display screen 12 may include light of a plurality of different wavebands. For example, the light emitted by the display screen 12 is white light. Therefore, to further reduce the costs of the test circuit and reduce occupation by the PCB 40 in the electronic device, the display screen of the electronic device for displaying is reused as the light-emitting assembly 521.

In this case, the optical proximity sensor 522 may be disposed in the non-display region of the display screen 12, or disposed below the display screen 12. During detection, the finger of the user only needs to cover the optical proximity sensor 522 and a part of the display region of the display screen 12.

It may be understood that when the display screen is reused as the light-emitting assembly 521, a display drive chip may control the display screen to emit an optical signal of a first preset color, where the optical signal of the first preset color may include light in at least two different bands.

For example, at the light-emitting stage T01, the display screen 12 may emit an optical signal of the first preset color according to a drive signal sent by the display drive chip. The optical signal of the first preset color may be a white light signal. The white light signal includes the first optical signal and the second optical signal. The first optical signal is, for example, a red light signal, and the second optical signal is, for example, a green light signal. When the optical signal emitted by the display screen 12 illuminates the skin tissue of the fingertip of the user, the first collection channel formed by the first photosensitive component 52211 and the first analog-to-digital converter 52221 collects ambient light and reflected and/or scattered light of the red light signal and converts the optical signal into a digital signal, the processing unit 5223 processes the digital signal, to obtain an integral value D5, the second collection channel formed by the second photosensitive component 52212 and the second analog-to-digital converter 52222 collects ambient light and reflected and/or scattered light of the green light signal and converts the optical signal into a digital signal, and the processing unit 5223 processes the digital signal, to obtain an integral value D7.

At the non-light-emitting stage T02, according to control of the display drive chip, the display screen does not emit light; the first collection channel formed by the first photosensitive component 52211 and the first analog-to-digital converter 52221 collects only ambient light and converts an optical signal into a digital signal; the processing unit 5223 processes the digital signal to obtain an integral value D6; the second collection channel formed by the second photosensitive component 52212 and the second analog-to-digital converter 52222 collects only ambient light and converts an optical signal into a digital signal; and the processing unit 5223 processes the digital signal to obtain an integral value D8. After one pulse cycle ends, the processing unit 5223 calculates a difference between the integral value D5 and the integral value D7, to obtain an integral value (including only the first optical signal component part) of the intensity of the first optical reference signal, and calculates a difference between the integral value D6 and the integral value D8, to obtain an integral value (including only the second optical signal component part) of the light intensity of the second optical reference signal, to exclude interference of the ambient light direct current component. The integral value of the light intensity of the first optical reference signal and the integral value of the light intensity of the second optical reference signal are then sent to the processor 51 for processing.

Descriptions are provided above by using an example in which the display drive chip controls the display screen to emit the optical signal of the first preset color, where the optical signal of the first preset color may include light in at least two different bands. In other optional embodiments of this application, the display drive chip may alternatively control the display screen to respectively emit only the first optical signal (for example, a red light signal) and the second optical signal (for example, a green light signal) at different moments. For a specific process of calculating the human body feature value according to the first optical signal and the second optical signal, refer to the foregoing content (corresponding to the content of the structure in FIG. 6 and the content of the sequence in FIG. 9), and details are not described herein again. Certainly, the light-emitting assembly emitting the broad-spectrum light source is not limited to the display screen 12. For example, the light-emitting assembly may alternatively be the flash light 54. The flash light 54 emits an optical signal of a second preset color. The optical signal of the second preset color may include light in at least two different bands. The optical signal of the second preset color is, for example, white light. That is, the flash light 54 not only has a supplementary lighting function, but also may be reused as a light-emitting assembly.

In this case, the optical proximity sensor 522 and the flash light 54 may be disposed on the same side of the mobile phone, and the optical proximity sensor 522 and the flash light 54 are disposed adjacent to each other. During detection, the finger of the user only needs to cover the optical proximity sensor 522 and the flash light 54.

For example, at the light-emitting stage T01, the flash light 54 may emit an optical signal of the second preset color according to the drive signal sent by the processing unit 5223 or the processor 51. The optical signal of the second preset color may be a white light signal. The white light signal includes the first optical signal and the second optical signal. The first optical signal is, for example, a red light signal, and the second optical signal is, for example, a green light signal. When an optical signal emitted by the flash light 54 illuminates the skin tissue of the fingertip of the user, the first photosensitive component 52211 and the first analog-to-digital converter 52221 collect ambient light and reflected and/or scattered light of the red light signal and convert an optical signal into a digital signal, the processing unit 5223 processes the digital signal, to obtain the integral value D5, the second collection channel formed by the second photosensitive component 52212 and the second analog-to-digital converter 52222 collects ambient light and reflected and/or scattered light of the green light signal and converts an optical signal into a digital signal, and the processing unit 5223 processes the digital signal to obtain the integral value D7.

At the non-light-emitting stage T02, the flash light 54 does not emit light, the first collection channel formed by the first photosensitive component 52211 and the first analog-to-digital converter 52221 collects only ambient light and converts an optical signal into a digital signal, the processing unit 5223 processes the digital signal to obtain the integral value D6, the second photosensitive component 52212 and the second analog-to-digital converter 52222 collects only ambient light and converts an optical signal into a digital signal, and the processing unit 5223 processes the digital signal to obtain the integral value D8. After one pulse cycle ends, the optical proximity sensor 522 calculates a difference between the integral value D5 and the integral value D7, to obtain an integral value (including only the first optical signal component part) of the light intensity of the first optical reference signal, and calculates a difference between the integral value D6 and the integral value D8, to obtain an integral value (including only the second optical signal component part) of the light intensity of the second optical reference signal, to exclude interference of the ambient light direct current component. The integral value of the light intensity of the first optical reference signal and the integral value of the light intensity of the second optical reference signal are then sent to the processor 51 for processing.

It should be noted that descriptions are provided above by using an example in which the optical proximity sensor includes a plurality of collection channels, and the plurality of collection channels can collect and process optical signals in different bands at the same time, but this does not constitute a limitation on this application, provided that a sensor in which a plurality of collection channels can be formed and can collect and the plurality of collection channels can process optical signals in different bands at the same time falls within the protection scope of this application.

In conclusion, the existing PPG module is replaced with the light-emitting assembly and the optical proximity sensor. Because the optical proximity sensor is widely applied and has a low price, costs of the test circuit can be reduced. In addition, because the collection unit, the analog-to-digital converter, and the processing unit are integrated inside the optical proximity sensor, a volume of the test circuit is small, and occupation of the volume of the electronic device by the test circuit is reduced. In addition, the test circuit detects reflected and/or scattered light of the first optical signal and the second optical signal, and detects ambient light. In this way, interference from external ambient light can be reduced, background noise can be filtered out, and detection accuracy can be improved. In addition, the photoelectric sensor according to this embodiment of this application may include a plurality of photosensitive components and a plurality of analog-to-digital converters. The plurality of photosensitive components and the plurality of analog-to-digital converters respectively form different collection channels. Therefore, the different collection channels can respectively collect optical signals having different wavelengths at the same time, and the tracking of the physiological change data is better. In this way, the detection accuracy can be further improved when the blood oxygen saturation, the heart rate, and the like are calculated based on the PPG signal. In addition, the test circuit can calculate the blood oxygen saturation, the heart rate, and the like based on signals in two different optical bands (for example, the first optical signal and the second optical signal), and can couple optical signals in other bands, for example, determine a PPG signal based on optical signals in three, four, five, or six optical bands, to calculate the human vital sign parameters such as a blood pressure, blood glucose, a perfusion index, a pulse variability index, and content of blood indicators such as carboxyhemoglobin and glycated hemoglobin.

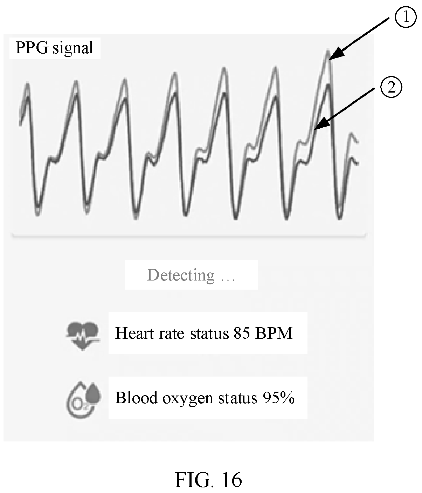

In addition, blood oxygen and heart rate tests were performed on the test circuit provided in this embodiment of this application. The test was conducted using a first optical signal as a red light signal and a second optical signal as an infrared light signal. With reference to FIG. 5, when a finger of a user touches the light-transmissive portion 21 of the rear cover 20 and remains still and quiet, the infrared light signal and red light signal emitted by the light-emitting assembly 521 are emitted through the light-transmissive portion 21 and illuminate a fingertip of the user. The infrared light signal and red light signal pass through the skin tissue of the fingertip and are then reflected to the optical proximity sensor 522. The optical proximity sensor 522 converts the light into an electrical signal and obtains a stable infrared light PPG signal and red light PPG signal based on the electrical signal, as shown in FIG. 16. FIG. 16 is a diagram of an interface of a test result of blood oxygen and a heart rate obtained based on a test circuit according to an embodiment of this application. In FIG. 16, an arrow ① points to the infrared signal and an arrow ② points to the red light PPG signal. It can be learned from the interface, based on the infrared light PPG signal and red light PPG signal obtained by the test circuit provided in this embodiment of this application, a heart rate status is 85 BPM and a blood oxygen status is 95%. Accuracy of the blood oxygen and heart rate test is basically the same as that of a pulse oximeter. That is, the electronic device 100 using the test circuit provided in this embodiment of this application can replace medical equipment to detect health status of the user in real time, and has low costs and high detection accuracy.

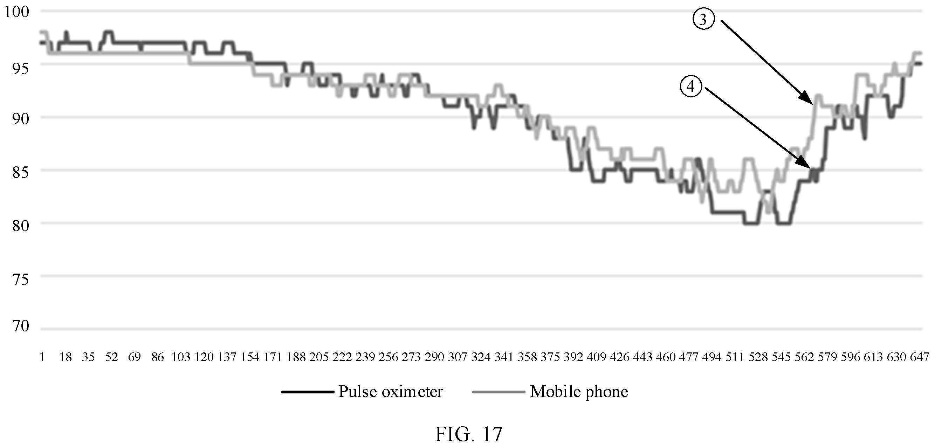

In addition, oxygen decreasing and increasing monitoring tests were conducted on the test circuit provided in this embodiment of this application. For example, three electronic devices using the test circuit in this application were tested, and each electronic device underwent simulated oxygen decreasing and increasing tests of six persons, and simulated oxygen decreasing and increasing tests were conducted on six persons using a medical pulse oximeter. An oxygen decreasing and increasing monitoring comparison chart is shown in FIG. 17. FIG. 17 only shows a comparison chart of oxygen decreasing and increasing test data for one person using both the electronic device with the test circuit provided in this embodiment of this application and the medical pulse oximeter. A pattern is the same for the other people. Therefore, the comparison charts of oxygen decreasing and increasing test data for the other people are not shown. In FIG. 17, an arrow (3) points to an oxygen decreasing and increasing test data curve obtained by the electronic device using the test circuit in this application, and an arrow (4) points to an oxygen decreasing and increasing test data curve obtained by the medical pulse oximeter. In FIG. 17, a horizontal axis represents sampling points, that is, a point is recorded every preset time, such as a point is recorded every 10 seconds, and a vertical axis represents a blood oxygen value. The tests show that the oxygen decreasing and increasing test data curve obtained based on the test circuit provided in this embodiment of this application are basically consistent with the oxygen decreasing and increasing test data curve obtained based on the pulse oximeter. Moreover, a root mean square error (Root Mean Square Error, RMSE) value can be calculated from the oxygen decreasing and increasing test data obtained based on the test circuit provided in this embodiment of this application, as shown in Table 1. An average value of the test data (18 groups of root mean square errors in total) of six persons corresponding to each of three electronic devices is obtained. The average value of the root mean square errors is, for example, 2.42%, and meets the FDA medical standard (the standard value RMSE is 3.5%).

**Table 1**

| Person | Electronic device 1 | Electronic device 2 | Electronic device 3 | |
|--------|---------------------|---------------------|---------------------|---|
| 1 | 0.022314623 | 0.021382642 | 0.018011919 | |
| 2 | 0.022516288 | 0.019476559 | 0.020437721 | |
| 3 | 0.019940576 | 0.020007551 | 0.018425296 | |
| 4 | 0.032570745 | 0.032798744 | 0.023094011 | |
| 5 | 0.029408993 | 0.046820077 | 0.025615159 | |
| 6 | 0.020110062 | 0.015690703 | 0.023908658 | |

(continued)

| Person | Electronic device 1 | Electronic device 2 | Electronic device 3 | |
|---|---|---|---|---|
| Average value | 0.024476881 | 0.026029379 | 0.021582127 | 0.0242 |

In addition, an embodiment of this application provides an electronic device. The electronic device further includes: a memory and a processor, where the memory is coupled to the processor; and the memory stores program instructions, and the program instructions, when executed by the processor, enable the electronic device to perform the foregoing related steps, to implement the test method in the foregoing embodiment.

For example, when the optical proximity sensor 522 includes the processing unit 5223, the processing unit 5223 sends the integral value of the light intensity of the first optical reference signal and the integral value of the light intensity of the second optical reference signal to the processor. The processor obtains an integral value of the light intensity of the first optical reference signal within a time period. That is, a plurality of duty cycles T0 within the time period correspond to integral values of light intensities of a plurality of first optical reference signals, and the integral values of the light intensities of the plurality of first optical reference signals may be stored in the memory. The integral values of the light intensities of the plurality of first optical reference signals may form a curve. The curve can represent an intensity of a red light signal (namely, a red light PPG signal). Moreover, the processor obtains the integral value of the light intensity of the second optical reference signal within a time period. That is, a plurality of duty cycles T0 within the time period correspond to the integral values of light intensities of the plurality of second optical reference signals. The integral values of the light intensities of the plurality of second optical reference signals may be stored in the memory. The integral values of the light intensities of the plurality of second optical reference signals may form a curve, and the curve can represent an intensity of an infrared light signal (namely, an infrared light PPG signal). The processor may separate an alternating current AC signal and a direct current DC signal of the red light and separate an alternating current AC signal and a direct current DC signal of the infrared light based on the red light PPG signal and the infrared light PPG signal that are stored in the memory, to obtain a ratio PI(RED) of the alternating current AC signal to the direct current DC signal of the red light and a ratio PI(IR) of the alternating current AC signal to the direct current DC signal of the infrared light, determine an R value based on PI(RED) and PI(IR), and further determine human body features such as the heart rate and the blood oxygen saturation.

For example, when the processing unit 5223 is a processor having a specific processing function in the electronic device, that is, the processor is the processing unit 5223, the processor directly obtains the integral value of the light intensity of the first optical reference signal within the time period. That is, the plurality of duty cycles T0 within the time period correspond to the integral values of the light intensities of the plurality of first optical reference signals, and the integral values of the light intensities of the plurality of first optical reference signals may be stored in the memory. The integral values of the light intensities of the plurality of first optical reference signals may form a curve, and the curve can represent the intensity of the red light signal (that is, the red light PPG signal). In addition, the processor directly obtains the integral value of the light intensity of the second optical reference signal within the time period, that is, the plurality of duty cycles T0 within the time period correspond to the integral values of the light intensities of the plurality of second optical reference signals, and may store the integral values of the light intensities of the plurality of first optical reference signals in the memory. The integral values of the light intensities of the plurality of second optical reference signals may form a curve, and the curve can represent the intensity of the infrared light signal (that is, the infrared light PPG signal). The processor may separate an alternating current AC signal and a direct current DC signal of the red light and separate an alternating current AC signal and a direct current DC signal of the infrared light based on the red light PPG signal and the infrared light PPG signal that are stored in the memory, to obtain a ratio PI(RED) of the alternating current AC signal to the direct current DC signal of the red light and a ratio PI(IR) of the alternating current AC signal to the direct current DC signal of the infrared light, determine an R value based on PI(RED) and PI(IR), and further determine human body features such as the heart rate and the blood oxygen saturation.

In addition, an embodiment of this application further provides a computer-readable storage medium. The computer-readable storage medium stores computer instructions, and the computer instructions, when run on an electronic device, enable the electronic device to perform the foregoing related steps, to implement the test method in the foregoing embodiment.

The embodiments further provide a computer program product, and the computer program product, when run on an electronic device, enables the electronic device to perform the foregoing related steps, to implement the test method in the foregoing embodiment.

In addition, an embodiment of this application further provides a chip (which may alternatively be a component or module). The chip may include one or more processing circuits and one or more transceiver pins. The transceiver pin and the processing circuit communicate with each other through an internal connection path. The processing circuit performs the foregoing related method steps, to implement the test method in the foregoing embodiment, to control a receive pin to receive a signal, and control a emit pin to emit a signal.

In addition, it may be learned from the foregoing descriptions that, the electronic device, the computer-readable storage medium, the computer program product, or the chip provided in embodiments of this application are all configured to perform the corresponding method provided above. Therefore, for beneficial effects that can be achieved by the electronic device, the computer-readable storage medium, the computer program product, or the chip, refer to the beneficial effects of the corresponding method provided above. Details are not described herein again.

The foregoing embodiments are merely used to describe the technical solutions of this application, but are not intended to limit the technical solutions. Although this application is described in detail with reference to the foregoing embodiments, it should be understood by a person of ordinary skill in the art that modifications can be made to the technical solutions described in the foregoing embodiments, or equivalent replacements can be made to some technical features in the technical solutions; and these modifications or replacements do not cause the essence of corresponding technical solutions to depart from the scope of the technical solutions in embodiments of this application.

**Claims**

1. A test circuit, comprising:

   a light-emitting assembly, configured to emit at least two types of optical signals, wherein the at least two types of optical signals comprise a first optical signal and a second optical signal; and the first optical signal and the second optical signal have different wavelengths; and
   an optical proximity sensor, configured to collect a first optical reference signal and a second optical reference signal that are reflected and/or scattered by a human body, wherein the first optical reference signal and the second optical reference signal are used for calculating a human body feature value.

2. The test circuit according to claim 1, wherein the optical proximity sensor comprises:

   a collection unit, configured to collect the first optical reference signal and the second optical reference signal, and separately convert the first optical reference signal and the second optical reference signal into electrical signals, wherein the electrical signals are analog signals; and
   an analog-to-digital converter, connected to the collection unit, and configured to convert the analog signals into digital signals, wherein the digital signals are used for calculating the human body feature value.

3. The test circuit according to claim 2, wherein the light-emitting assembly comprises a first light-emitting unit and a second light-emitting unit, the collection unit comprises a first photosensitive component and a second photosensitive component, the first light-emitting unit is configured to emit the first optical signal, the first photosensitive component is configured to collect the first optical reference signal, the second light-emitting unit is configured to emit the second optical signal, and the second photosensitive component is configured to collect the second optical reference signal.

4. The test circuit according to claim 3, wherein the optical proximity sensor comprises the first light-emitting unit or the second light-emitting unit.

5. The test circuit according to any one of claims 2 to 4, further comprising a processing unit, wherein the processing unit is connected to the analog-to-digital converter and configured to obtain, based on the digital signals, an integral value of a light intensity of the first optical reference signal within a first time period and an integral value of a light intensity of the second optical reference signal within a second time period, and the integral values are used for calculating the human body feature value.

6. The test circuit according to claim 5, wherein the light-emitting assembly emits the first optical signal and the second optical signal according to a duty cycle.

7. The test circuit according to claim 6, wherein the duty cycle comprises at least a first stage and a second stage; the first stage and the second stage both comprise at least one pulse cycle, and the pulse cycle comprises a light-emitting stage at which working is performed in a first level state and a non-light-emitting stage at which working is performed in a second level state;

   the light-emitting assembly is configured to emit the first optical signal at the light-emitting stage of the first stage, and emit the second optical signal at the light-emitting stage of the second stage;
   the optical proximity sensor is configured to collect a third optical signal at the light-emitting stage of the first stage, and collect a fourth optical signal at the non-light-emitting stage of the first stage; and is further configured to collect a fifth optical signal at the light-emitting stage of the second stage, and collect a sixth optical signal at the non-light-emitting stage of the second stage;

the processing unit is configured to obtain the integral value of the light intensity of the first optical reference signal based on the third optical signal and the fourth optical signal; and is further configured to obtain the integral value of the light intensity of the second optical reference signal based on the fifth optical signal and the sixth optical signal;

the first time period is the first stage, and the second time period is the second stage; and

the third optical signal is determined based on ambient light and reflection and/or scattering of the first optical signal, the fourth optical signal is determined based on the ambient light, the fifth optical signal is determined based on the ambient light and reflection and/or scattering of the second optical signal, and the sixth optical signal is determined based on the ambient light.

8. The test circuit according to claim 7, wherein the collection unit comprises a first photosensitive component and a second photosensitive component, the first photosensitive component and the analog-to-digital converter form a first collection channel, and the second photosensitive component and the analog-to-digital converter form a second collection channel;

the first collection channel is configured to collect the third optical signal at the light-emitting stage of the first stage, and collect the fourth optical signal at the non-light-emitting stage of the first stage; and

the second collection channel is configured to collect the fifth optical signal at the light-emitting stage of the second stage, and collect the sixth optical signal at the non-light-emitting stage of the second stage.

9. The test circuit according to claim 7 or 8, wherein the first stage and the second stage both comprise N pulse cycles, wherein $N \geq 2$, and N is a positive integer.

10. The test circuit according to claim 5, wherein a duty cycle comprises at least one pulse cycle, and the pulse cycle comprises a light-emitting stage at which working is performed in a first level state and a non-light-emitting stage at which working is performed in a second level state;

the light-emitting assembly is configured to emit the first optical signal and the second optical signal at the light-emitting stage;

the optical proximity sensor is configured to collect a seventh optical signal and an eighth optical signal at the light-emitting stage, and collect a ninth optical signal and a tenth optical signal at the non-light-emitting stage;

the processing unit is configured to obtain the integral value of the light intensity of the first optical reference signal based on the seventh optical signal and the ninth optical signal; and is further configured to obtain the integral value of the light intensity of the second optical reference signal based on the eighth optical signal and the tenth optical signal;

the first time period and the second time period are a same time period, and the first time period is one duty cycle; and

the seventh optical signal is determined based on ambient light and reflection and/or scattering of the first optical signal, the eighth optical signal is determined based on the ambient light and reflection and/or scattering of the second optical signal, the ninth optical signal is determined based on the ambient light, and the tenth optical signal is determined based on the ambient light.

11. The test circuit according to claim 10, wherein the collection unit comprises a first photosensitive component and a second photosensitive component, and the analog-to-digital converter comprises a first analog-to-digital converter and a second analog-to-digital converter; the first photosensitive component and the first analog-to-digital converter form a first collection channel, and the second photosensitive component and the second analog-to-digital converter form a second collection channel;

the first collection channel is configured to collect the seventh optical signal at the light-emitting stage, and collect the ninth optical signal at the non-light-emitting stage; and

the second collection channel is configured to collect the eighth optical signal at the light-emitting stage, and collect the tenth optical signal at the non-light-emitting stage.

12. The test circuit according to claim 10 or 11, wherein the duty cycle comprises N pulse cycles, wherein $N \geq 2$, and N is a positive integer.

13. The test circuit according to any one of claims 1 to 12, wherein the first optical signal comprises a red light signal, and the second optical signal comprises an infrared light signal.

14. The test circuit according to any one of claims 5 to 13, wherein the optical proximity sensor comprises the processing unit.

15. An electronic device, comprising the test circuit according to any one of claims 1 to 14.

16. The electronic device according to claim 15, further comprising a display screen, wherein the display screen is reused as the light-emitting assembly.

17. The electronic device according to claim 16, wherein the display screen comprises a display region and a non-display region surrounding the display region, and the optical proximity sensor is located in the non-display region.

18. The electronic device according to claim 15, comprising a display screen and a flash light that are disposed on opposite sides of the electronic device, wherein the optical proximity sensor and the flash light are disposed on a same side of the electronic device, the flash light is reused as the light-emitting assembly, and an optical signal emitted by the flash light comprises at least the first optical signal and the second optical signal.

19. A test method, applied to an electronic device, wherein the electronic device comprises a plurality of duty cycles, the duty cycle comprises at least one pulse cycle, and the pulse cycle comprises a light-emitting stage at which working is performed in a first level state and a non-light-emitting stage at which working is performed in a second level state; and the method comprises:

   emitting a first optical signal and a second optical signal, and collecting a seventh optical signal and an eighth optical signal at the light-emitting stage, wherein the first optical signal and the second optical signal have different wavelengths, the seventh optical signal is determined based on ambient light and reflection and/or scattering of the first optical signal, and the eighth optical signal is determined based on the ambient light and reflection and/or scattering of the second optical signal;
   collecting a ninth optical signal and a tenth optical signal at the non-light-emitting stage, wherein the ninth optical signal is determined by the ambient light, and the tenth optical signal is determined by the ambient light; and
   obtaining an integral value of a light intensity of a first optical reference signal based on the seventh optical signal and the ninth optical signal, and obtaining an integral value of a light intensity of a second optical reference signal based on the eighth optical signal and the tenth optical signal.

20. An electronic device, comprising: a memory and a processor, wherein the memory is coupled to the processor; and the memory stores program instructions, and the program instructions, when executed by the processor, enable the electronic device to perform the test method according to claim 19.

Absorption spectra of different types of hemoglobin

FIG. 1

FIG. 2

AA'

21    20

30

51 521 522 40

10

52

50

FIG. 3

Approaching object

Isolating
foam

Detector    Light source

FIG. 4

FIG. 5

FIG. 6

FIG. 7

FIG. 8

FIG. 9

The flicker light
source lights

The
flicker
light
source
does
not
light

Working
sequence of a
flicker light
source

Working
sequence of a
light-emitting
assembly

T01　T02　T01　T02　T01　T02　T01　T02

T1　T2　T1　T2

T0　T0

FIG. 10

T01　T01　T01　T01　T01　T01　T01　T01

T02　T02　T02　T02　T02　T02　T02　T02

T1　T2　T1　T2

T0　T0

FIG. 11

FIG. 12

FIG. 13

Working
sequence of a
second optical
signal

Working
sequence of a
first optical
signal

T01 | T02 | T01 | T02 | T01 | T02 | T01 | T02

T0 | T0 | T0 | T0

FIG. 14

Working
sequence
of a
second
optical
signal

Working
sequence
of a first
optical
signal

T01 | T01 | T01 | T01 | T01 | T01 | T01 | T01
T02 | T02 | T02 | T02 | T02 | T02 | T02 | T02

T0 | T0 | T0 | T0

FIG. 15

FIG. 16

FIG. 17

EP 4 781 905 A1

## INTERNATIONAL SEARCH REPORT

| International application No. |
|---|
| **PCT/CN2024/107911** |

| A. | CLASSIFICATION OF SUBJECT MATTER |
|---|---|
| | A61B5/024(2006.01)i |

According to International Patent Classification (IPC) or to both national classification and IPC

| B. | FIELDS SEARCHED |
|---|---|

Minimum documentation searched (classification system followed by classification symbols)

IPC:A61B5

Documentation searched other than minimum documentation to the extent that such documents are included in the fields searched

Electronic data base consulted during the international search (name of data base and, where practicable, search terms used)

CNABS, CNTXT, CNKI: 发光, 光源, 红光, 红外光, 光电检测, 环境光, 关闭, 截止, 积分, 血氧, 光电容积, 脉搏, 血流; VEN, USTXT, WOTXT, EPTXT, IEEE, ElsevierScience, SpringerLink: light, red, infrared, photoelectric, close, stop, integral, saturatio n oxygen, , photocapacitance, pulse, blood

| C. | DOCUMENTS CONSIDERED TO BE RELEVANT |
|---|---|

| Category* | Citation of document, with indication, where appropriate, of the relevant passages | Relevant to claim No. |
|---|---|---|
| X | US 2018055429 A1 (SEIKO EPSON CORP.) 01 March 2018 (2018-03-01) description, paragraphs 25-69, and figures 1-12 | 1-6, 13-15, 18 |
| Y | US 2018055429 A1 (SEIKO EPSON CORP.) 01 March 2018 (2018-03-01) description, paragraphs 25-69, and figures 1-12 | 7-20 |
| Y | CN 115561826 A (WUHAN SILICON INTEGRATED CO., LTD.) 03 January 2023 (2023-01-03) description, paragraphs 6-9 | 7-20 |
| Y | CN 113576474 A (GUANGDONG OPPO MOBILE TELECOMMUNICATIONS CORP., LTD.) 02 November 2021 (2021-11-02) description, paragraphs 28-45, and figures 1-4 | 16-17 |
| Y | CN 104865563 A (HANGZHOU SILAN MICROELECTRONICS CO., LTD.) 26 August 2015 (2015-08-26) description, paragraphs 35-89, and figures 1-6 | 7-20 |

| ✓ Further documents are listed in the continuation of Box C. | ✓ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **10 October 2024** | **18 October 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**EP 4 781 905 A1**

## INTERNATIONAL SEARCH REPORT

<table>
<tr><td colspan="2"></td><td>International application No.<br>**PCT/CN2024/107911**</td></tr>
</table>

**C.    DOCUMENTS CONSIDERED TO BE RELEVANT**

| Category* | Citation of document, with indication, where appropriate, of the relevant passages | Relevant to claim No. |
|---|---|---|
| Y | CN 110604558 A (GUANGDONG OPPO MOBILE TELECOMMUNICATIONS CORP., LTD.) 24 December 2019 (2019-12-24)<br>description, paragraphs 27-49, and figures 1-2 | 16-17 |
| X | US 2021330208 A1 (COVIDIEN LP) 28 October 2021 (2021-10-28)<br>description, paragraphs 15-84, and figures 1-5 | 1-4, 13-15, 18 |
| X | CN 112806972 A (GUANGDONG OPPO MOBILE TELECOMMUNICATIONS CORP., LTD.) 18 May 2021 (2021-05-18)<br>description, paragraphs 30-77, and figures 1-6 | 1-4, 13-15, 18 |
| A | US 2015190063 A1 (BIOVOTION AG) 09 July 2015 (2015-07-09)<br>entire document | 1-20 |

Form PCT/ISA/210 (second sheet) (July 2022)

**INTERNATIONAL SEARCH REPORT**
Information on patent family members

International application No.

**PCT/CN2024/107911**

| Patent document cited in search report | | | Publication date (day/month/year) | Patent family member(s) | | | Publication date (day/month/year) |
|---|---|---|---|---|---|---|---|
| US | 2018055429 | A1 | 01 March 2018 | US | 10660552 | B2 | 26 May 2020 |
| | | | | JP | 2018029869 | A | 01 March 2018 |
| | | | | JP | 7052191 | B2 | 12 April 2022 |
| CN | 115561826 | A | 03 January 2023 | CN | 115561826 | B | 11 August 2023 |
| CN | 113576474 | A | 02 November 2021 | | None | | |
| CN | 104865563 | A | 26 August 2015 | CN | 104865563 | B | 03 October 2017 |
| CN | 110604558 | A | 24 December 2019 | | None | | |
| US | 2021330208 | A1 | 28 October 2021 | EP | 4142587 | A1 | 08 March 2023 |
| | | | | US | 11633116 | B2 | 25 April 2023 |
| | | | | WO | 2021222007 | A1 | 04 November 2021 |
| CN | 112806972 | A | 18 May 2021 | WO | 2021098694 | A1 | 27 May 2021 |
| | | | | CN | 112806972 | B | 07 April 2023 |
| US | 2015190063 | A1 | 09 July 2015 | US | 9526431 | B2 | 27 December 2016 |
| | | | | KR | 20140084183 | A | 04 July 2014 |
| | | | | KR | 101860605 | B1 | 23 May 2018 |
| | | | | WO | 2013056379 | A1 | 25 April 2013 |
| | | | | EP | 2768391 | A1 | 27 August 2014 |
| | | | | EP | 2768391 | B1 | 01 May 2019 |

Form PCT/ISA/210 (patent family annex) (July 2022)

**EP 4 781 905 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311344325